(19) Europäisches Patentamt
European Patent Office
Office européen des brevets

(11) EP 2 647 634 A1

(12) EUROPEAN PATENT APPLICATION

(43) Date of publication:
**09.10.2013 Bulletin 2013/41**

(21) Application number: **12382133.2**

(22) Date of filing: **02.04.2012**

(51) Int Cl.:
***C07D 403/04*** (2006.01) ***A61K 31/506*** (2006.01)
***A61P 25/00*** (2006.01) ***A61P 3/10*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NOSCIRA, S.A.**
**28760 Tres Cantos, Madrid (ES)**

(72) Inventors:
- **Medina Padilla, Miguel**
  **E-28760 Tres Cantos - Madrid (ES)**
- **Dominguez Correa, Juan Manuel**
  **E-28760 Tres Cantos - Madrid (ES)**
- **Fuertes Huerta, Ana**
  **E-28760 Tres Cantos - Madrid (ES)**
- **Palomo Nicolau, Francisco**
  **E-28760 Tres Cantos - Madrid (ES)**
- **Lopez Ogalla, Javier**
  **E-28760 Tres Cantos - Madrid (ES)**
- **Herrero Santos, Susana**
  **E-28760 Tres Cantos - Madrid (ES)**
- **Alonso Cascon, Mercedes**
  **E-28760 Tres Cantos - Madrid (ES)**
- **Alonso Gordillo, Diana**
  **E-28760 Tres Cantos - Madrid (ES)**
- **Rubio Arrieta, Laura**
  **E-28760 Tres Cantos - Madrid (ES)**

(74) Representative: **ABG Patentes, S.L.**
**Avenida de Burgos, 16D**
**Edificio Euromor**
**28036 Madrid (ES)**

# (54) Indole-pyrimidine derivatives and their therapeutic uses

(57) The present invention relates to new indole-pyrimidine derivatives of Formula (I), a process for obtaining them, a pharmaceutical composition comprising said compounds and their use in the treatment of several disease including cognitive, neurodegenerative or neurological diseases or conditions, diabetes, inflammatory and autoimmune diseases and cardiovascular disorders.



Printed by Jouve, 75001 PARIS (FR)

## Description

## FIELD OF THE INVENTION

The present invention is related to a new family of compounds, namely indole-pyrimidine derivatives of Formula (I); said compounds for use as a medicament; a pharmaceutical composition comprising said compounds; said compounds for use in the treatment of certain diseases or conditions; the use of said compounds as a reactive in an *in vitro* biological assay requiring inhibition of GSK-3, CDK5 or CK1ε; the use of said compounds in the preparation of a medicament for the treatment of certain diseases or conditions; and a method of treating a disease, comprising administering said compounds.

## BACKGROUND OF THE INVENTION

### Cyclin-dependent kinase 5 (Cdk5)

Cyclin- dependent kinase 5 (Cdk5) is a proline- directed serine/ threonine protein kinase with a growing number of identified substrates, such as β- Amyloid precursor protein (β- APP), presenilin- 1, tau protein or neurofilaments (NFs) [Nat. Rev., Mol. Cell Biol., 2001, 2, 749- 759. *A decade of CDK5.* Dhavan R. & Tsai L.H.; Journal of Neurochemistry, 2004, 88, 1313- 1326. *Cyclin- dependent kinase- 5 in neurodegeneration.* Shelton S.B. & Johnson G. V. W.; Cell Mol Neurobiol, 2008, 28, 351- 369. *An Unusual Member of the Cdk Family: Cdk5. Dhariwala F.A. & Rajadhyaksha M.S.*] . Cdk5 does not play a role in cell division but plays a pivotal role in the development of the central nervous system (CNS) [Nat. Rev., Mol. Cell Biol., 2001, 2, 749- 759. A decade of CDK5. Dhavan R. & Tsai L.H.] . Cdk5 functions beyond neurodevelopment and may play a role in learning and memory, in dopamine signaling in the basal forebrain, in the migration and diferentiation of neurons during histogenesis of the brain, in regulation of the cytoskeletal elements, membrane transport, synaptic function and during neuronal differentiation and axonal extension. Therefore, CDK5 likely plays a role in synaptic plasticity, cellular motility and adhesion, drug addiction and neurodegeneration [Biochimica et Biophysica Acta 1697 (2004) 137- 142. Cdk5, a therapeutic target for Alzheimer's disease?. Tsai L.H., Lee M.S. & Cruz J*;* Cell Mol Neurobiol, 2008, 28, 351- 369. An Unusual Member of the Cdk Family: Cdk5. Dhariwala F.A. & Rajadhyaksha M.S.; J. Comp. Neurol., 1999, 415, 218- 229. Callosal axon guidance defects in p35 (- /- ) mice. Kwon Y.T., Tsai L.H. & Crandall J.E.; Journal of Neurochemistry, 2004, 88, 1313- 1326*. Cyclin- dependent kinase- 5 in neurodegeneration. Shelton S.B. & Johnson G. V. W.*] .

In order for Cdk5 to become active, it must form a heterodimer with p35, p25 (the truncated form of p35), p39 or p29 (the truncated form of p39) [Journal of Neurochemistry, 2004, 88, 1313-1326. Cyclin-dependent kinase-5 in neurodegeneration. Shelton S.B. & Johnson G.V.W.]. Both p35 and p39 have short half-life *in vivo* and are subject to ubiquitin-mediated proteosome degradation indicating that Cdk5 activity is normally tightly regulated. Several neurotoxic conditions, including ischemic brain damage, oxidative stress, excitotoxicity and β-amyloid peptide treatment of primary neurons, were shown to induce calpain-mediated p35 cleavage and p25 production. p25, which is neurotoxic, contains all the elements necessary for Cdk5 activation. It efficiently activates Cdk5 both *in vitro* and *in vivo.* p25 and p35 have distinct properties: p35 has a very short half-life but p25 is stable and has a much longer half-life; p35 is localized to the membranes but p25 localizes to the cell soma and nucleus. As such, generation of p25 disrupts the normal regulation of Cdk5 by causing prolonged activation and mislocalization of Cdk5 and resulting in hyperphosphorylation of available substrates [Biochimica et Biophysica Acta 1697 (2004) 137-142. Cdk5, a therapeutic target for Alzheimer's disease?. Tsai L.H., Lee M.S. & Cruz J.].

Although Cdk5 is expressed in most tissues, its activity is found predominantly in post-mitotic neurons [Development, 2003, 119, 1029-1040. Activity and expression pattern of cyclin-dependent kinase 5 in the embryonic mouse nervous system. Tsai L. H. *et al.].* The reason for the localized activity of Cdk5 is that its activators, p35 and p39, are expressed almost exclusively in the CNS [Nature, 1994, 371, 423-426. A brain-specific activator of cyclin-dependent kinase 5. Lew *et al.;* Nature, 1994, 3 71, 419-423. p35 is a neural-specific regulatory subunit of cyclin-dependent kinase 5. Tsai *et al.;* FEBS Lett., 1994, 355, 35-40. Precursor of cdk5 activator, the 23 kDa subunit of tau protein kinase II: its sequence and developmental change in brain. Uchida *et al.;* J. Biol. Chem., 1995, 270, 26897-26903. An isoform of the neuronal cyclin-dependent kinase 5 (Cdk5) activator. Tang *et al.;* Neuroscience, 2003, 118, 323-334. The cyclin-dependent kinase 5 activator, p39, is expressed in stripes in the mouse cerebellum Jeong *et al.;* Cell. Mol. Biol. Lett., 2003, 8, 546,547. The role of cyclin-dependent kinase 5 in brain development and degeneration Tsai L.H.]. Although Cdk5 is present predominantly in the nervous system, this enzyme has been implicated to be an important molecule in other systems as well. Since its discovery over a decade ago an increasing number of the Cdk5 substrates have been reported making it one of the most versatile kinases. Cdk5 is also known to play an important role in other non-neuronal functions like myogenesis, haematopoesis, reproduction, prostate cancer, differentiation of lens epithelial and corneal cells, gene-transcription, senescence, insulin secretion, apoptosis, exocytosis/endocytosis, hormonal regulation and wound healing

and migration [for a review, see Cell Mol Neurobiol, 2008, 28, 351-369. An Unusual Member of the Cdk Family: Cdk5. Dhariwala F.A. & Rajadhyaksha M.S.*;* J.Comp. Neurol., 1999, 415, 218-229. Callosal axon guidance defects in p35 (-/-) mice. Kwon Y.T., Tsai L.H. & Crandall J. E.].

The Cdk5 kinase is activated in cultured neurons following p25 overexpression, and leads to tau hyperphosphorylation and apoptosis. *In vitro,* certain neurotoxic insults, including exposure to β-amyloid (Aβ), trigger p25 accumulation, Cdk5 activation, and neuronal death, which is averted by pharmacological cdk inhibitors. Moreover, extracellular Aβ deposits induce Cdk5 activation and tau hyperphosphorylation in mutant APP transgenic mice [American Journal of Pathology, 2004, 165, 3, 843-853. Cyclin-Dependent Kinase Inhibitors Attenuate Protein Hyperphosphorylation, Cytoskeletal Lesion Formation, and Motor Defects in Niemann-Pick Type C Mice. Zhang M. *et al.;* J. Neurosci. Res., 2002, 69, 362-372. p35/ Cdk5 pathway mediates soluble amyloid-beta peptide-induced tau phosphorylation in vitro. Town T. *et al*.].

Cdk5 has a major activity phosphorylating tau in brain [J. Biochem. (Tokyo), 1997, 121, 179-188. Physiology and pathology of tau protein kinases in relation to Alzheimer's disease. Imahori K. & Uchida T.]. Cdk5, or downstream kinases, can phosphorylate tau prior to, or independently of, aggregation [Neuron, 2003, 38, 555-565. Cdk5 Is a Key Factor in Tau Aggregation and Tangle Formation In Vivo. Noble W. *et al*.]. Many Cdk5 phosphorylation sites on tau have been identified. Ser202, Thr205, Thr212, Ser235, Ser396 and Ser404, all Ser/Thr-Pro sites, are the most likely to be physiologically relevant, as several groups have identified them as Cdk5 sites when tau is phosphorylated *in vitro* [Journal of Neurochemistry, 2004, 88, 1313-1326. Cyclin-dependent kinase-5 in neurodegeneration. Shelton S.B. & Johnson G.V.W; Neuron, 2003, 38, 555-565. Cdk5 Is a Key Factor in Tau Aggregation and Tangle Formation In Vivo. Noble W. et al.]. Although most of the studies have been carried out *in vitro,* there is some evidence of tau phosphorylation by Cdk5 *in situ* and *in vivo.* Hence, Cdk5 may play a role in the process of NFT formation. Increased Cdk5 activity leads to insoluble aggregation and fibrillization of endogenous tau in the cortex and hippocampus of p25 Tg mice. Furthermore, Cdk5 may indirectly regulate the kinases and phosphatases that act on tau. Cdk5 has been shown to modulate the activity of several protein kinases involved in tau hyperphosphorylation [Neuron, 2003, 40, 471-483. Aberrant Cdk5 Activation by p25 Triggers Pathological Events Leading to Neurodegeneration and Neurofibrillary Tangles. Cruz J.C. et al.; Journal of Neurochemistry, 2004, 88, 1313-1326. Cyclin-dependent kinase-5 in neurodegeneration. Shelton S.B. & Johnson G.V.W].

Additionally, has been demonstrated that Cdk5 inhibitors can reduce β- amyloid (Aβ) level in the mouse brain [J. Neurosci., 2008, 28, 2624- 2632. Interplay between Cyclin- Dependent Kinase 5 and Glycogen Synthase Kinase 3β Mediated by Neuregulin Signaling Leads to Differential Effects on Tau Phosphorylation and Amyloid Precursor Protein Processing. Wen, Y. *et al*.] since Aβ generation correlates with enhanced Cdk5 activity. Cdk5 has been shown to phosphorylate APP on Thr668 in the cytoplasmic domain. It is reported that T668- phosphorylated APP is up- regulated in AD brain tissues. Moreover the observations that p25 is elevated in AD brain tissue and in an animal model for β-amyloid and that p25 can be induced by Aβ peptide treatment of primary neurons indicate that p35 to p25 cleavage may be a downstream event of β- amyloid toxicity [Biochimica et Biophysica Acta 1697 (2004) 137- 142. Cdk5, a therapeutic target for Alzheimer's disease?. Tsai L.H., Lee M.S. & Cruz J. ] .

Cdk5/p25 might also interact with other pathways such as glycogen synthetase kinase 3beta (GSK3beta) and c-JUN kinase. Therefore, Cdk5, and other kinases such as glycogen synthase kinase 3β (GSK3β), have been identified as prime candidates for AD pathogenesis because they both generate disease associated phospho-epitopes on tau, and they colocalize with filamentous tau aggregates in the brains of patients and in a transgenic mouse model of tauopathy. Cdk5 and GSK3 also regulate Aβ production in *vivo* [J. Neurosci., 2008, 28, 2624- 2632. Interplay between Cyclin-Dependent Kinase 5 and Glycogen Synthase Kinase 3β Mediated by Neuregulin Signaling Leads to Differential Effects on Tau Phosphorylation and Amyloid Precursor Protein Processing. Wen, Y. *et al.].*

***Pathologies related to Cyclin-dependent kinase 5 (Cdk5)***

Dysregulation of cyclin-dependent kinases (Cdks), specifically Cdk5, and cytoskeletal protein hyperphosphorylation characterizes a subset of human neurodegenerative diseases, including Alzheimer's disease [Biochimica et Biophysica Acta 1697 (2004) 137-142. Cdk5, a therapeutic target for Alzheimer's disease?. Tsai L.H., Lee M.S. & Cruz J.], amyotrophic lateral sclerosis [Neurosci. Lett., 1998, 245, 45-48. Cyclin-dependent kinase-5 is associated with lipofuscin in motor neurones in amyotrophic lateral sclerosis. Bajaj *et al*.], Niemann-Pick Type C (NPC) [Neurobiol Dis, 2002, 11, 285-97. Niemann-Pick disease type C yields possible clue for why cerebellar neurons do not form neurofibrillary tangles. Bu B. *et al*.], alcohol-induced neurodegeneration [Neurosci. Lett., 2001, 308, 173-176. Ethanol induced changes in cyclin-dependent kinase-5 activity and its activators, P35, P67 (Munc-18) in rat brain Rajgopal Y. & Vemuri M.C.], diffuse Lewy body disease [Am. J. Pathol., 1995, 147, 1465-1476. Cortical and brainstem-type Lewy bodies are immunoreactive for the cyclin-dependent kinase 5. Brion J.P. & Couck A.M.], Parkinson's disease [Proc Natl Acad Sci U S A, 2003, 100, 13650-13655. Cyclin-dependent kinase 5 is a mediator of dopaminergic neuron loss in a mouse model of Parkinson's disease. Smith P.D. *et al*.], progressive supranuclear palsy [Neurology, 2002, 58, 589-592. Increase of cdk5

is related to neurofibrillary pathology in progressive supranuclear palsy. Borghi R. *et al.*], frontotemporal dementia and parkinsonism linked to chromosome 17 (FTDP-17) and other tauopathies [J Biol Chem., 2009, 15, 284(20), 13422-33. Familial FTDP-17 missense mutations inhibit microtubule assembly-promoting activity of tau by increasing phosphorylation at Ser202 in vitro. Han D. *et al.*], amnestic mild cognitive impairment [Neurochem Res., 2007, 32(4-5), 655-62. Regional expression of key cell cycle proteins in brain from subjects with amnestic mild cognitive impairment. Sultana R. & Butterfield D.A.], Down syndrome [Neuroscience, 2008, 22, 156(1), 99-106. Abnormal expression of synaptic proteins and neurotrophin-3 in the Down syndrome mouse model Ts65Dn. Pollonini G. *et al.*], Huntington disease [J Neurosci., 2008, 1, 28(40), 10090-101. *Dopaminergic and glutamatergic signaling crosstalk in Huntington's disease neurodegeneration: the role of p25/cyclin-dependent kinase 5. Paoletti P. et al.*], multiple sclerosis [J Biol Chem., 2004, 31, 279(53), 55833-9. Hyperphosphorylation and aggregation of tau in experimental autoimmune encephalomyelitis. Schneider A. *et al.*], and stroke [Proc Natl Acad Sci USA, 2000, 97, 10254-10259. Cyclin-dependent kinases as a therapeutic target for stroke. Osuga H. *et al.*].

Apart from neurodegenerative diseases, a series of disorders with potential Cdk5 involvement are brain ischemia [Nature Neuroscience, 2003, 6, 1039-1047. Cdk5 activation induces hippocampal CA1 cell death by directly phosphorylating NMDA receptors. Wang J. *et al.*], acute neuronal injury [J Neurochem, 2004, 88(6), 1313-1326. Cyclin-dependent kinase-5 in neurodegeneration. Shelton S.B. & Johnson G.V.W.], cocaine addiction [Nature, 2001, 410, 376-380. Effects of chronic exposure to cocaine are regulated by the neuronal protein Cdk5. Bibb J.A. *et al.*], electroconvulsive seizures [J. Neurosci., 2000 20, 8965-8971. Induction of Cyclin-Dependent Kinase 5 in the Hippocampus by Chronic Electroconvulsive Seizures: Role of ΔFosB. Chen J. *et al.*], fetal alcohol syndrome [Neurosci. Lett., 2001, 308, 173-176. Ethanol induced changes in cyclin-dependent kinase-5 activity and its activators, P35, P67 (Munc-18) in rat brain Rajgopal Y. & Vemuri M.C.], focal cortical dysplasia [Dev Neurosci., 2008, 30(1-3), 96-104. The potential role of cyclin-dependent kinase 5 in focal cortical dysplasia. Sen A. *et al.*], hereditary canine spinal muscular atrophy [J. Neuropathol. Exp. Neurol., 1998, 57(11), 991-1099. Alterations in Cyclin-Dependent Protein Kinase 5 (CDK5) Protein Levels, Activity and Immunocytochemistry in Canine Motor Neuron Disease. Green S. *et al.*], inclusion body myositis [Neurology 1999, 53, 1671-1676. Aberrant expression of cyclin-dependent kinase 5 in inclusion body myositis Nakano S. *et al.*], multiple system atrophy [J. Neuropathol. Exp. Neurol., 1998, 57, 690-698. Cyclin-dependent kinase 5 and mitogen-activated protein kinase in glial cytoplasmic inclusions in multiple system atrophy. Nakamura S. *et al.*], peripheral nerve injury [Neuroreport., 2002, 13, 243-247. Induction of Cdk5 activity in rat skeletal muscle after nerve injury. Fu W.Y. *et al.*], anxiety and stress [Eur Neuropsychopharmacol., 2010, 20(6), 388-397. Previous stress exposure enhances both anxiety-like behaviour and p35 levels in the basolateral amygdala complex: Modulation by midazolam. Bignante E.A. *et al.*], epilepsy [Synapse, 2009, 63(11), 1017-1028. Gene expression analysis on anterior temporal neocortex of patients with intractable epilepsy. Xi Z. Q. *et al.*], prion diseases [J Neurosci., 2009, 8, 29(27), 8743-8751. Phosphorylation of Prion Protein at Serine 43 Induces Prion Protein Conformational Change. Paresa N. *et al.*], ataxia [Mol Cell Biochem., 2008, 318(1-2), 7-12. Tyrosine hydroxylase expression and Cdk5 kinase activity in ataxic cerebellum. Cheung K.J. *et al.*], squizophrenia [Neuroreport., 2006, 17(18), 1903-5. Expression of p25 impairs contextual learning but not latent inhibition in mice. Mizuno K. *et al.*], pain [Cell Cycle, 2006, 5:6, 585-588. Cdk5: A New Player in Pain Signaling. Tej K. *et al.*], diabetic neuropathy [Rinsho Shinkeigaku., 1999. 39(1), 87-9. New trend in pathogenesis of diabetic neuropathy. Yasuda H.], and diabetes mellitus [Mini Rev Med Chem., 2007, 7(10), 1070-4. Cyclin-dependent kinase 5 (Cdk5): a potential therapeutic target for the treatment of neurodegenerative diseases and diabetes mellitus. Wei F.Y. & Tomizawa K.].

**Glycogen synthase kinase-3 (GSK-3)**

Glycogen synthase kinase-3 (GSK-3) is a serine/threonine protein kinase comprised of α and β isoforms that are each encoded by distinct genes (Chemistry & Biology, 2000, 7(10), 793-803. Selective small molecule inhibitors ofglycogen synthase kinase-3 modulate glycogen metabolism and gene transcription. Coghlan *et al.;* Curr. Opinion Genetics Dev., 2000, 10(5), 508-514. GSK3, a master switch regulating cell-fate specification and tumorigenesis. Kim, L. & Kimmel, A.R.). GSK-3 plays critical roles in development, metabolic homeostasis, neuronal growth and differentiation, cell polarity, cell fate and in modulation of apoptotic potential.

***Pathologies related to Glycogen synthase kinase-3 (GSK-3)***

Deregulation (usually increase) of GSK-3 activity is believed to play a role in different and important disorders like neurodegenerative disorders [Physiol. Rev., 2004, 84, 361-84. Role of tau protein in both physiological and pathological conditions. Ávila, J *et al.*], cardiovascular disease [Circ Res., 2009, 104(11), 1240-52; Role of glycogen synthase kinase-3beta in cardioprotection. Juhaszova M. *et al.;* Circ J., 2009, 73(7), 1184-92. GSK-3beta, a therapeutic target for cardiomyocyte protection. Miura T. & Miki T.], diabetes [Trends. Mol. Med., 2002, 8, 126-32. Glycogen synthase kinase 3: an emerging therapeutic target. Eldar-Finkelman, H.] or viral infections [Virus Res., 2008, 132, 160-73. Residues in human respiratory syncytial virus P protein that are essential for its activity on RNA viral synthesis. Asenjo, A. *et al.*].

Regarding neurodegenerative disorders and other CNS pathologies, GSK- 3 dysregulation has been related to Alzheimer's disease [Current Pharmaceutical Design, 2010, 16 (25) : 2790- 2798. Glycogen Synthase Kinase- 3 (GSK- 3) Inhibitors For The Treatment of Alzheimer's Disease. Medina M. & Ávila J. *;* Brain Res Bull., 2009, 80 (4- 5), 248- 50. The role of GSK3 in Alzheimer disease. Hernández F. *et al.*] *,* mild cognitive impairment [J Psychiatr Res., 2011, 45 (2), 220- 4. Increased platelet GSK3B activity in patients with mild cognitive impairment and Alzheimer's disease. Forlenza O.V. *et al.*] *,* Parkinson's disease [Neuroscience Letters 2009, 449 (2), 103- 107. Glycogen synthase kinase-3beta is associated with Parkinson's disease. Masahiro N. & Hideaki H. ] *,* frontotemporal dementia [Arch. Neurol., 2008, 65, 1368- 74. Association of GSK3B with Alzheimer disease and frontotemporal dementia.. Schaffer, B. *et al.*] *,* frontotemporal lobar degeneration associated with Pick bodies [Int J Alzheimers Dis., 2011, 2011, 352805. Functional implications of glycogen synthase kinase- 3- mediated tau phosphorylation. Hanger D.P. & Noble W. ] *,* Pick's disease, progressive supranuclear palsy, subacute sclerosing panencephalitis, postencephalitic parkinsonism, dementia pugilistica, guam parkinsonism- dementia complex, corticobasal degeneration, argyrophilic grain disease, familial frontotemporal dementia and parkinsonism linked to chromosome 17 due to mutations in the tau gene (FTDP- 17- tau) and other tauopathies *[*Frontiers in Molecular Neurosciences, 2011, 4 (24) : 1- 10. Modulation of GSK- 3 as a therapeutic strategy on taupathologies. Medina, M., Garrido, J.J. & Wandosell, F. *;* Brain Research Reviews, 2000, 33, 95- 130. Tau protein isoforms, phosphorylation and role in neurodegenerative disorders. Buée, L. *et al.*] *,* AIDS associated dementia [J Neuroimmune Pharmacol., 2007, 2 (1), 93- 96*. Glycogen synthase kinase 3 beta (GSK- 3 beta) as a therapeutic target in neuroAIDS. Dewhurst S. et al.*] *,* Huntington's disease [J Biol Chem., 2002, 277 (37), 33 791- 8. Glycogen synthase kinase- 3beta inhibitors prevent cellular polyglutamine toxicity caused by the Huntington's disease mutation. Carmichael J. *et al.*] *,* Lewy body disease [Neuropathology, 2003, 23 (3), 199- 202. Glycogen synthase kinase- 3beta phosphorylates synphilin- 1 in vitro. Tanji K. *et al.*] *,* bipolar disorder [Neurosci Biobehav Rev., 2007, 31 (6), 920- 931; GSK- 3 is a viable potential target for therapeutic intervention in bipolar disorder. Roew M.K. *et al.;* Bipolar Disord., 2002, 4 (2), 137- 144. Glycogen Synthase Kinase- 3β, mood stabilizers, and neuroprotection. Li X. *et al.*] *,* depression *[*J Pharmacol Sci., 2009, 110 (1), 14- 28. Lithium and neuropsychiatric therapeutics: neuroplasticity via glycogen synthase kinase- 3beta, beta- catenin, and neurotrophin cascades. Wada A. ] *,* schizophrenia [Drug News Perspect., 2007, 20 (7), 437- 45. The role of glycogen synthase kinase- 3beta in schizophrenia. Koros E. & Dorner- Ciossek C. *;* Trends Neurosci., 2007, 30 (4), 142- 9. Schizophrenia as a GSK- 3 dysregulation disorder. Lovestone S. *et al.],* epilepsy [J Neurochem., 1999, 72 (3), 1327- 30. The mood- stabilizing agent valproate inhibits the activity of glycogen synthase kinase- 3. Chen G. *et al.*] *,* mood disorders [Curr Drug Targets., 2006, 7 (11), 1421- 34. Glycogen synthase kinase- 3 (GSK3) in psychiatric diseases and therapeutic interventions. Jope R. S. & Roh M.S. ] *,* autism [Proc Natl Acad Sci U S A., 2008, 105 (4), 1333- 8. Role of GSK3 beta in behavioral abnormalities induced by serotonin deficiency. Beaulieu J.M. *et al.*] *,* attention deficit hyperactivity disorder [Proc Natl Acad Sci U S A., 2004, 101 (14), 5099- 104. Lithium antagonizes dopamine-dependent behaviors mediated by an AKT/ glycogen synthase kinase 3 signaling cascade. Beaulieu J.M. *et al.*], Down's syndrome [FASEB J., 2008, 22 (9), 3224- 33. Overexpression of Dyrk1A contributes to neurofibrillary degeneration in Down syndrome. Liu F. *et al.*], fragile X syndrome (FXS) [Biochem Pharmacol., 2010, 79 (4), 632- 46. Lithium ameliorates altered glycogen synthase kinase- 3 and behavior in a mouse model of fragile X syndrome. Yuskaitis C.J. *et al.*] *,* diseases associated with ischemia/ reperfusion and shock [Shock., 2007, 27 (2), 113- 23. Glycogen synthase kinase 3beta as a target for the therapy of shock and inflammation. Dugo L. *et al.*] *,* brain injury *[*Neurol Res., 2001, 23 (6), 588- 92. Different expression of glycogen synthase kinase- 3beta between young and old rat brains after transient middle cerebral artery occlusion. Sasaki C. *et al.*] *,* multiple sclerosis [Trends Immunol., 2010, 31 (1), 24- 31. Innate and adaptive immune responses regulated by glycogen synthase kinase- 3 (GSK3) . Beurel E. *et al.*] and other autoimmune and inflammatory diseases afflicting the CNS [J. Immunol., 2008, 181 (1), 338- 45. Lithium prevents and ameliorates experimental autoimmune encephalomyelitis. De Sarno P. *et al.*] *,* spinocerebellar ataxia type 1 [PLoS Med, 2007, 4 (5), 836- 847. Lithium therapy improves neurological function and hippocampal dendritic arborization in a spinocerebellar ataxia type 1 mouse model. Watase K. *et al.*] *,* cerebral bleeding for example, due to solitary cerebral amyloid angiopathy [Neuroscience., 2008, 153 (2), 414- 27. Accumulation of beta- amyloid in the brain microvessels accompanies increased hyperphosphorylated tau proteins following microsphere embolism in aged rats. Han F. *et al.*] *,* amyotrophic lateral sclerosis [Brain Res., 2008, 1196, 131- 139. Upregulation of GSK3β expression in frontal and temporal cortex in ALS with cognitive impairment (ALSci) . Yang W. *et al.],* prion disease *[*Biochem J., 2003, 15, 372 (Pt 1), 129- 36. Prion peptide induces neuronal cell death through a pathway involving glycogen synthase kinase 3. Pérez M. *et al.*] *,* Gerstman-Sträussler- Scheinker disease [BMC Infect Dis., 2010, 1, 10, 86. Changes of tau profiles in brains of the hamsters infected with scrapie strains 2 63 K or 139 A possibly associated with the alteration of phosphate kinases. Wang G.R. *et al.],* Hallervorden- Spatz disease and multiple systems atrophy [Cell Mol Neurobiol., 2008, 28 (1), 21- 33. Overexpressed alpha- synuclein regulated the nuclear factor- kappaB signal pathway. Yuan Y. et al.] or myotonic dystrophy [Cell Cycle. 2009, 8, 15, 2356- 9. GSK3beta- cyclin D3- CUGBP1- eIF2 pathway in aging and in myotonic dystrophy. Jin J. *etal.] .*

In addition to its possible relevance to prevent neurodegeneration, GSK3 inhibitors may also be useful to foster other forms of neuronal repair, including axon regeneration [J. Neurosci., 2008, 28, 8914-28. Inactivation of glycogen synthase kinase 3 promotes axonal growth and recovery in the CNS. Dill, J. *et al.*].

During the last few years, GSK- 3 has been identified as a regulator of many components of the immune system, suggesting it might be a plausible therapeutic target in inflammatory and autoimmune diseases, such as chronic inflammatory diseases including rheumatoid arthritis, inflammatory bowel disease and psoriasis [Eur J Biochem., 2001, 268 (19), 5001- 10. The role ofprotein phosphorylation in human health and disease. Cohen P. ] , arthritis [Clin. Immunol., 2006, 120, 57- 67. Glycogen synthase kinase- 3b inhibition attenuates the degree of arthritis caused by type II collagen in the mouse. Cuzzocrea, S. *et al*.] , peritonitis [Immunity, 2006, 24, 563- 574. IFN- g suppresses IL- 10 production and synergizes with TLR2 by regulating GSK3 and CREB/AP- 1 proteins. Hu, X. *et al.],* systemic inflammation, renal dysfunction and hepatotoxicity in endotoxemia [Crit. Care Med., 2005, 33, 1903- 1912. GSK- 3b inhibitors attenuate the organ injury/ dysfunction caused by endotoxemia in the rat. Dugo, L. *et al*.], asthma [Am JPhysiol Lung Cell Mol Physiol., 2009, 296 (2), L176- 84. Airway smooth muscle hyperplasia and hypertrophy correlate with glycogen synthase kinase- 3 (beta) phosphorylation in a mouse model of asthma. Bentley J.K. *et al*.] , sepsis [J. Biochem. Cell. Biol., 2005, 37, 2226- 2238. GSK- 3b inhibitors reduce protein degradation in muscles from septic rats and in dexamethasone treated myotubes. Int. Evenson, A.R. *et al*.] , colitis [Br. J. Pharmacol., 2006, 147, 575- 582. Reduction of experimental colitis in the rat by inhibitors of glycogen synthase kinase- 3b. Whittle, B.J. *et al.],* inflammation- induced organ injury caused by hemorrhage and resuscitation *[*Shock, 2006, 25, 485- 491. Glycogen synthase kinase- 3b inhibitors protect against the organ injury and dysfunction caused by hemorrhage and resuscitation. Dugo, L. *et al*.], inflammatory injury in chronic renal allograft disease [Am J Transplant., 2008, 8 (9), 1852- 63. Glycogen synthase kinase 3beta: a novel marker and modulator of inflammatory injury in chronic renal allograft disease. Gong R. *et al*.] or lupus [Int. J. Immunopharmacol., 1995, 17, 581- 592. Lithium chloride enhances survival of NZB/W lupus mice: influence of melatonin and timing of treatment. Lenz, S.P. *et al*.] .

Among cardiovascular disorders related to GSK-3 are heart disease [Circ. Res., 2002, 90, 1055-63. Glycogen synthase kinase-3beta: a novel regulator of cardiac hypertrophy and development. Hardt, S.E. & Sadoshima, J.], atherosclerosis [Am J Pathol., 2009, 174(1), 330-42. Valproate attenuates accelerated atherosclerosis in hyperglycemic apoE-deficient mice: evidence in support of a role for endoplasmic reticulum stress and glycogen synthase kinase-3 in lesion development and hepatic steatosis. Bowes A.J. *et al*.], hypertension [J. Clin. Invest., 2002, 109(3), 373-381. Fas receptor signaling inhibits glycogen synthase kinase 3β and induces cardiac hypertrophy following pressure overload. Badorff C. *et al*.], restenosis [Cardiovasc Res., 2010, Epub. Delayed Re-endothelialization with Rapamycin-coated Stents is Rescued by the Addition of a Glycogen Synthase Kinase 3 Beta Inhibitor. Ma X. et al.] or leukopenia [Gallicchio, V. S. (1991) in Lithium and the Cell, ed. Birch, N. J. (Academic, San Diego), pp. 185-198.].

Additional pathologies associated with GSK-3 are metabolic syndrome X [Curr Pharm Des., 2004, 10(10), 1105-37. Discovery and development of GSK3 inhibitors for the treatment of type 2 diabetes. Wagman A.S. *et al.],* hair loss [J Clin Invest., 2010, 120(2), 446-56. Neural Wiskott-Aldrich syndrome protein modulates Wnt signaling and is required for hair follicle cycling in mice. Lyubimova A. *et al*.], severe acute respiratory syndrome coronavirus [J Biol Chem., 2009, 284(8), 5229-39. Glycogen synthase kinase-3 regulates the phosphorylation of severe acute respiratory syndrome coronavirus nucleocapsid protein and viral replication. Wu C.H. *et al*.], cocaine addiction [J Neurochem., 2009, 111(6), 1357-68. Glycogen synthase kinase 3beta in the nucleus accumbens core mediates cocaine-induced behavioral sensitization. Xu C.M. *et al*.], bone loss [Life Sci., 2009, 85(19-20), 685-92. Inhibition of glycogen synthase kinase-3beta attenuates glucocorticoid-induced bone loss. Wang F.S. *et al.]* or glaucoma [J Clin Invest., 2008, 118(3), 1056-64. Increased expression of the WNT antagonist sFRP-1 in glaucoma elevates intraocular pressure. Wang W.H. *et al*.].

***GSK-3 inhibitors***

For a further review of GSK-3 inhibitors and their use as potential treatments for these pathologies, please reference to Nature Reviews, 2004, 3, 479-487. GSK3 inhibitors: development end therapeutic potential. Cohen, P. & Goedert, M.*;* Mini-Reviews in Medicinal Chemistry, 2009, 9(9), 1024-1029. GSK3 Inhibitors and Disease. Hernández, F. *et al.;* Curr. Opin. Drug Discov. Develop., 2008, 11(4), 533-543. Glycogen synthase kinase-3 (GSK-3) inhibitors reach the clinic. Medina, M. & Castro, A.*;* John Wiley & Sons, Inc., 2006. Glycogen Synthase Kinase 3 (GSK-3) and its inhibitors. Chapter 14. Eds: Martinez, A., Castro, A. & Medina, M.

Several GSK- 3 inhibitors like indirubines [J. Biol. Chem., 2001, 276, 251- 60. Indirubins inhibit glycogen synthase kinase- 3 beta and CDK5lp25, two protein kinases involved in abnormal tau phosphorylation in Alzheimer's disease. A property common to most cyclin- dependent kinase inhibitors?. Leclerc, S. *et al*.] , maleimides [Bioorg. Med. Chem. Lett., 2001, 11, 635- 9. 3- Anilino- 4- arylmaleimides: potent and selective inhibitors of glycogen synthase kinase- 3 (GSK- 3) . Smith, D. *et al*.] , 3- amino pyrazoles *[*Bioorg. Med. Chem. Lett., 2003, 13, 1581- 4. 5- arylpyrazolo [3, 4- b] pyridazines: potent inhibitors of glycogen synthase kinase- 3 (GSK- 3) . Witherington, J. et al.] , paullones *[*Eur. J. Biochem., 2000, 267, 5983- 94. Paullones are potent inhibitors of glycogen synthase kinase- 3beta and cyclin- dependent kinase 5/p25. Leost, M. *et al*.] , thiazoles [J. Biol. Chem., 2003, 278, 45937- 45. Structural insights and biological effects of glycogen synthase kinase 3- specific inhibitor AR- A014418. Bhat, R. *et al*.] or thiadiazolidinones [J. Med. Chem., 2002, 45, 1292- 9. First non- ATP competitive glycogen synthase kinase 3 beta (GSK- 3beta) inhibitors: thiadiazolidinones

(TDZD) as potential drugs for the treatment of Alzheimer's disease. Martinez, A. *et al.*] have been described.

**Casein kinase 1 (CK1)**

Casein kinase 1 (also refered to as CK1, CK-1 or CKI) is a serine/threonine specific protein kinase that is ubiquitously expressed in eukaryotic organisms [Ospina and Fernandez-Renart (1990). Biochim. Biophys. Acta 1052, 483; Tuazon and Traugh (1991). Adv. Second Messenger Phosphoprot.Res. 23, 123; Klimczak and Cashmore (1993). Biochem. J 293 (Pt. 1), 283; Walczak et al. (1993). Biochem. J 296 (Pt. 3), 729; Pulgar et al. (1996). Eur. J. Biochem. 242, 519; Peters et al. (1999). Nature 401, 345; Liu et al. (2003). Plant J. 36, 189]. The CK1 family of monomeric protein kinases is found in eukaryotic organisms from yeast to human. Mammals have seven family members (sometimes referred to as isoforms, but encoded by distinct genes): Ckiα, Ckiβ, Ckilγ1, Ckilγ2, Ckilγ3, Ckiδ and Ckiε. Members of the CK1 family are constitutively active and can be isolated as active enzymes from many different cell types, in several subcellular compartments, including the plasma membrane, cytosol, and nucleus [for a review, see Knippschild et al. (2005). Cellular Signalling, 17: 675-689; Flajolet et al. (2007). PNAS. 104 (10), 4159-4164; Pérez D.I. et al. Med Res Rev. (2010)].

In addition, CK1 is involved in diverse biological functions, such as membrane transport [Lampe P.D. & Lau A.F. (2004) . Int J Biochem Cell Biol, 36: 1171- 1186] regulation of DNA repair [Knippschild U. et al. (2005) . Onkologie 2005; 28: 508- 514] , cellular morphology [Robinson L.C. et al. (1993) . Mol Cell Biol; 13: 2870- 2881] , modulation of the Wnt/ bcatenin and Hedgehog pathway during development [Price M.A. (2006) . Genes Dev., 20: 399- 410] and regulation of circadian rhythms [Eide E.J. & Virshup D.M. (2001) . Chronobiol Int. 18: 389- 398; Ebisawa T. (2007) . J Pharmacol Sci, 103: 150- 154] . In mammals, CK1δ and ε phosphorylate PERIOD proteins, one of the key components of the circadian clock, promote their degradation [Meng Q.J. et al. (2008) . Neuron, 58: 78- 88] . Thereby, the overexpression of CK1δ and/or ε should result in the acceleration of the mammalian clock in the brain and periphery (for review, see Gross and Anderson (1998), Cell Signalling, 10: 699- 711; Knippschild et al. (2005) . Cellular Signalling, 17: 675- 689) .

Its subcellular localization and compartmentalization is an important factor in the regulation of kinase-substrate interactions [Wang et al. (1992). Mol. Biol. Cell 3, 275; Vancura et al. (1994). J Biol. Chem. 269, 19271; Ho et al. (1997). Proc. Natl. Acad. Sci. U. S. A. 94, 581]. Another mechanism to affect CK1 activity is its inhibitory autophosphorylation, which occurs especially in the C-terminal domain of CK1 [DeMaggio et al. (1992). Proc. Natl. Acad. Sci. U. S. A. 89, 7008; Graves et al. (1993). J. Biol. Chem. 268, 6394; Carmel et al. (1994). J. Biol. Chem. 269, 7304; Fish et al. (1995). J Biol. Chem. 270, 14875; Zhai et al. (1995). J Biol.Chem. 270, 12717]. CK1δ, ε and γ3 have large C-terminal domains, which have been suggested to function as pseudosubstrates, thereby inhibiting their own kinase activity [(Fish et al. (1995). J Biol. Chem. 270, 14875; Graves and Roach (1995). J. Biol. Chem. 270, 21689; Zhai et al. (1995). J Biol.Chem. 270, 12717; Graves et al. (1993). J Biol. Chem. 268, 6394; Longenecker et al. (1998). Acta Crystallogr., D Biol. Crystallogr. 54 (Pt 3), 473], although *in vivo* phosphatases, such as protein phosphatases 1 and 2A (PP1 and PP2A), keep it constitutively active in many cases [Rivers A. et al. (1998). J Biol. Chem. 273, 15980; Gietzen KF, Virshup DM (1999) JBiol Chem., 274:32063-32070].

Among the ever increasing number of CK1 substrates are enzymes, transcription factors, splice factors, cytoskeleton proteins, receptors, membrane-associated proteins and cell signalling proteins [Gross & Anderson (1998). Cell Signal, 10: 699-711; Vancura et al. (1994). J. Biol. Chem. 269, 19271].

In neurons, CK1 phosphorylates a variety of proteins including transcription factors, as well as certain synaptic vesicle proteins [Issinger (1993). Pharmacol Ther 59:1-30; Gross et al. (1995). J Cell Biol 130:711-724]. CK1δ and CK1ε, both expressed predominantly in the brain [Gross & Anderson (1998). Cell Signalling 10: 699-711], have been implicated in several important brain processes, including but not limited to: dopamine signalling (phosphorylation of dopamine- and cAMP-regulated phosphoprotein of 32 kDa), circadian rhythm (phosphorylation of protein Period), and neurotransmitter receptor phosphorylation [Desdouits et al. (1995). Proc Natl Acad Sci USA 92:2682-2685; Singh et al. (1995). J Neurochem 64:1420-1423; Kloss et al. (2001). Neuron 30:699-706]. In addition, CK1 is implicated in cell division, apoptosis or in the regulation of the Wnt pathway [Knippschild et al. (2005). Cellular Signalling, 17: 675-689]. Sakanaka showed that CkIε is an important regulator of β-catenin in the Wnt pathway and is a component of these complexes. CKIε is a positive regulator of the Wnt pathway and is a possible functional link between upstream signals and the intracellular axin signalling complex that regulates β-catenin [Sakanaka et al. (1999). PNAS, 96(22): 12548-12552].

During recent years, several studies have highlighted the importance of CK1 in neurodegenerative diseases, especially in tauopathies, such as Alzheimer's disease. Several isoforms of the CKI family (α, δ, and ε) are found in filamentous and granulovacuolar lesions within Alzheimer's brains, primarily within the hippocampus [Kuret et al. (1997). J. Neurochem. 69, 2506-2515; Ghoshal et al. (1999). Am. J. Pathol. 155, 1163-1172; Jellinger K.A. & Bancher C. (1998). J. Neural Transm Suppl 54: 77-95]. In addition, the levels of these CK1 isoforms in disease lesions are elevated severalfold over the levels found in controls [Vierlhaber & Virshup (2001). IUBMB Life, 51: 73-78]. Moreover, β-amyloid (Aβ) protein stimulates CK1 activities, contributing to abnormal protein phosphorylation in AD [Chauhan A. et al. (1993). Brain Res., 629: 47-52].

Neurofibrillary tangles are characteristic lesions found in the brain of AD patients. These tangles are composed of paired helical filaments (PHFs), and the major component of the latter is the microtubule-associated protein tau [Grundke-Igbal I. et al. (1986). J. Biol. Chem. 261, 6084-6089*].* CK1 isoforms are capable of phosphorylating tau in vitro at sites found in filamentous-tau [Singh et al. (1994). Mol. Cell. Bioch. 131: 181-9*;* Singh et al. (1995). JNeurochem, 64: 1420-3]. Moreover CK1 is one of the few known GSK3-priming kinases, which also include PKA, CK2, Cdk5, and DYRK1A *[*Meijer et al. (2004). Trends Pharmacol Sci., 25:471-480]. It has also been shown that CK1 is an upstream regulator of Cdk5, another protein kinase implicated in AD [Liu et al. (2001). Proc Natl Acad Sci USA, 98:11062-11068*;* Walter et al. (2001). J Biol Chem 276:14634-14641].

AD is also associated with the accumulation of the neurotoxic peptide Aβ, which is produced by sequential cleavage of the amyloid precursor protein (APP) by the aspartyl protease β-secretase and the presenilin-dependent protease γ-secretase. Overexpression of constitutively active CK1ε leads to an increase of Aβ peptide production. CK1-specific inhibitors significantly reduce endogenous Aβ peptide production by selectively interfering with amyloid precursor protein (APP) γ-cleavage. CK 1 acts directly or indirectly on the γ-secretase activity to selectively interfere with the APP γ-cleavage site but not at the Notch/APP ε-cleavage site [Flajolet et al. (2007). PNAS. 104 (10), 4159-4164*].* CK1 has also been shown to phosphorylate presenilin 2 *[*Walter et al. (1998) Biochemistry 37:5961—5967]. Additionally, CK-1 or a CK-1-like kinase is involved in the phosphorylation of BACE *in vivo* [Walter et al. (2001). J. Biol. Chem. 276 (18), 14634—14641].

Recently, it was reported that all major CK1 isoforms are expressed in the striatum and the cortex, and that CK1 regulates glutamatergic synaptic transmission mediated by NMDA receptors. Given the central role of NMDA receptors in several physiological and pathological processes in the striatum and throughout the CNS, CK1 may be an important regulatory enzyme in normal states, such as movement- and motivation- related behaviors [Chergi K. et al. (2005) . J. Neurosci., 25 (28) : 6601— 6609] .

***Pathologies related to Casein kinase 1***

CKIα, δ, and ε play an important role in neurodegenerative diseases, especially in tauopathies like Alzheimer's disease [Knippschild et al. (2005) . Cellular Signalling, 17: 675- 689*],* Parkinson's disease, dementia with Lewy body disease (DLB), Lewy body variant of Alzheimer disease (LBVAD) and multiple systems atrophy (MSA) *[*Waxman E.A. & Giasson B.I. (2008) . J Neuropathol Exp Neurol., 67 (5) : 402—416] *,* progressive supranuclear palsy, frontotemporal dementia and Parkinsonism linked to chromosome 17 (FTDP- 17), corticobasal degeneration, Pick disease pallido-ponto- nigral degeneration, Parkinsonism dementia complex of Guam, subacute sclerosing panencephalitis, postencephalitic parkinsonism, dementia pugilistica and Pick's disease [Singh, T.J. et al. (1995) . J. Neurochem., 64 (3*) .* Pérez D.I. et al. Med Res Rev. (2010*) ;* Schwab C. et al. (2000) . Neurobiology of Aging 21, 503- 510*;* Ferrer I. et al. (2005) . Current Alzheimer Research, 2, 3- 18*;* Churcher, I. (2006) . Current Topics in Medicinal Chemistry, 6 (6) : 579- 595*],* frontotemporal lobar degeneration with ubiquitinated inclusions (FTLD- U) and in dementias, including dementias of mixed and degenerative origin [Engelberg H. (2004) . Dement. Geriatr. Cogn. Disord. 18_278- 298*;* Larson E.B. et al. Annu. Rev. Publ. Health, 13: 431- 49*],* amyotrophic lateral sclerosis (ALS) *[*Hasegawa M. et al. (2008) . Ann Neurol.; 64 (1) : 60—70] .

Apart from neurodegenerative diseases, a series of disorders with potential CK-1 involvement are age-related diseases [Maclaine N.J. & Hupp T.R. (2009). Aging (Albany NY). 1(5) 490—502], Down syndrome [Schwab C. et al. (2000). Neurobiology of Aging 21, 503-510*],* stimulant drugs addiction [Veenstra-VanderWeele J. et al. (2006). Neuropsychopharmacology, 31, 1056—1063], febrile seizures [Yinan M. et al. (2004). Neuroscience Letters. 368(1), 2-6], epilepsy [Shinoda S. et al. (2003). Journal of Neurochemistry, 86, 460—469], circadian disorders such as depression, seasonal affective disorder, and metabolic disorders [Walton K.M. et al. (2009). The Journal of Pharmacology and Experimental Therapeutics, 330:430—439].

Additionally, Sakurai et al. have shown several lines of evidence that CK1ε plays a key role in the maintenance of neuropathic pain induced by spinal nerve injury. Thus, CK1 isoforms expressed in central and peripheral nervous system might display region and individual cell- specific regulation of synaptic transmission in normal and pathological states. Furthermore CK1 inhibitor was found to be effective in blocking excitatory synaptic responses in the spinal dorsal horn and neuropathic pain symptoms. These results suggest that CK1ε plays important physiological roles in neuropathic pain signalling, which makes it a useful target for analgesic drug development [Sakurai E. et al. (2009) . Molecular Pain, 5: 74] .

**Phosphorylation of Tau protein**

Tau is the major neuronal microtubule associated protein (MAP); the other two known MAPs in neurons are the high molecular weight MAPs, MAP1 and MAP2. Although tau protein is found in neurons, it can be expressed in glial cells, mainly in pathological conditions, and it is possible to detect tau mRNA and proteins in several peripheral

tissues such as heart, kidney, lung, muscle, pancreas, testis, as well as in fibroblasts. Tau is coded by a single gene on chromosome 17 but is expressed in several molecular isoforms that are generated by alternative splicing of its mRNA. In human brain, the alternative splicing of the mRNA results in six molecular isoforms.

Tau proteins bind to spectrin and actin filaments. Through these interactions, tau proteins may allow microtubules to interconnect with other cytoskeletal components such as neurofilaments and may restrict the flexibility of the microtubules. There is also evidence that tau proteins interact with cytoplasmic organelles. Such interactions may allow for binding between microtubules and mitochondria. The tau N-terminal projection domain also permits interactions with the neural plasma membrane. Thus, tau may act as a mediator between microtubules and plasma membrane.

Tau is a phosphoprotein and its biological activity is regulated by the degree of its phosphorylation. Normal brain tau contains 2—3 moles of phosphate per mole of the protein, which appears to be optimal for its interaction with tubulin and the promotion of microtubule assembly. In addition to phosphorylation, the alternative splicing also affects the biological activity of tau. In turn, tau proteins provide the microtubule with its own identity and physical characters (rigidity, length, stability, interactive capacity with other organelles). Therefore, by regulating microtubule assembly, tau proteins have a role in modulating the functional organization of the neuron, and particularly in axonal morphology, growth, and polarity.

For a review, please see Acta Neuropathol., 2009, 118(1), 53-69. Mechanisms of tau-induced neurodegeneration. Iqbal, K. *et al.;* Brain Research Reviews, 2000, 33, 95 —130. Tau protein isoforms, phosphorylation and role in neurodegenerative disorders. Buée, L. *et al.*]

***Tau protein and disease***

Tau protein is abnormally hyperphosphorylated in Alzheimer's disease (AD) brain and in this form, it is the major protein subunit of the paired helical filaments (PHF) and straight filaments (SF) forming neurofibrillary tangles (NFT), neuropil threads, and plaque dystrophic neurites in AD. Tau, which is phosphorylated at over 38 serine/threonine residues in AD, is a substrate for several protein kinases. Among these kinases, glycogen synthase kinase-3 (GSK-3), cyclin dependent protein kinase-5 (cdk5), protein kinase A (PKA), calcium and calmodulin-dependent protein kinase-II (CaMKII), casein kinase-1 (CK-1), mitogen activated protein (MAP) kinase ERK 1/2, and stress-activated protein kinases (SAPKs) have been most implicated in the abnormal hyperphosphorylation of tau.

The tau polymerized into NFT is apparently inert and neither binds to tubulin nor promotes its assembly into microtubules. Furthermore, the AD cytosolic abnormally hyperphosphorylated tau (AD Ptau) does not bind to tubulin and promote microtubule assembly, but instead it inhibits assembly and disrupts microtubules. This toxic property of the pathological tau involves the sequestration of normal tau by the diseased protein. The AD P-tau also sequesters the other two major neuronal MAPs, MAP1 A/B and MAP2.

Neurofibrillary degeneration of abnormally hyperphosphorylated tau not only occurs in AD brain but is also seen in a family of related neurodegenerative diseases, called tauopathies. In every one of these tauopathies, the neurofibrillary changes are made up of abnormally hyperphosphorylated tau and their occurrence in the neocortex is associated with dementia. In AD brain, all of the six tau isoforms are hyperphosphorylated and aggregated into PHF. In frontotemporal dementia with Parkinsonism- linked to chromosome 17 and tau pathology (FTDP- 17- tau), several missense mutations in tau co- segregate with the disease [see Acta Neuropathol., 2009, 118 (1), 53- 69. Mechanisms of tau- induced neurodegeneration. Iqbal, K. *et al.]* .

Among these diseases characterized by abnormal hyperphosphorylation of tau are Parkinson's disease-related dementia [Mov Disord., 2009, 15, 24(15), 2203-10. Cerebrospinal tau, phospho-tau, and beta-amyloid and neuropsychological functions in Parkinson's disease. Compta, Y. *et al.*], AIDS associated dementia [Neurology, 2005, 65(9), 1490-2. CSF amyloid beta42 and tau levels correlate with AIDS dementia complex. Brew, B.J. *et al.*], Down syndrome and senile dementia [Neuropathol. Appl. Neurobiol., 1984, 10, 185—207. Alzheimer's presenile dementia, senile dementia of Alzheimer type and Down's syndrome in middle age form an age related continuum of pathological changes, Mann, D.M.A. *et al.*], mild cognitive impairment [J Neurol Neurosurg Psychiatry, 2009, 80(9), 966-75. CSF phosphorylated tau in the diagnosis and prognosis of mild cognitive impairment and Alzheimer's disease: a meta-analysis of 51 studies. Mitchell , A.J.], Pick disease [Arch. Pathol. Lab. Med., 2006, 130, 1063-1066. Pick disease. A Brief Overview. Frederick, J.], Lewy body dementia [Int Rev Neurobiol., 2009, 84, 215-28. Lewy body dementia. Hanson, J.C. & Lippa, C.F.], Niemann—Pick disease [Brain, 1985, 118, 119—129 Neurofibrillary tangles in Niemann-Pick disease type C. Love, S. *et al.*], dementia with argyrophilic grains [Brain., 2008, 131(6), 1416-32. Argyrophilic grain disease. Ferrer, I. *et al.*], frontotemporal lobar degeneration, including frontotemporal dementia, progressive apraxia and progressive non-fluent aphasia [Acta Neuropathol., 2007, 114(1), 31-8. Frontotemporal lobar degeneration: clinical and pathological relationships. Snowden, J. *et al.*], frontotemporal dementia with Parkinsonism-linked to chromosome 17 and tau pathology [Nature, 1998, 393, 702—705. Association of missense and 50-splice-site mutations in tau with the inherited dementia FTDP-17. Hutton, M. *et al.*], multiple system tauopathy with presenile dementia [Brain, 2008, 131(1), 72-89. The tauopathy associated with mutation +3 in intron 10 of Tau: characterization of the MSTD family. Spina, S. *et al.*], dementia pugilistica

or chronic traumatic encephalopathy (CTE) and head trauma [Acta Neuropathol., 1992, 85, 23—30. Differential distribution of neurofibrillary tangles in the cerebral cortex of dementia pugilistica and Alzheimer's disease cases. Hof, P.R. *et al.;* J Neuropathol Exp Neurol., 2009, 68(7), 709-35. Chronic traumatic encephalopathy in athletes: progressive tauopathy after repetitive head injury. McKee, A.C. *et al.*], progressive supranuclear palsy [Lancet Neurol., 2009, 8(3), 270-9. Progressive supranuclear palsy: clinicopathological concepts and diagnostic challenges. Williams, D.R. & Lees, A.J.], postencephalitic parkinsonism *[*Acta Neuropathol., 2009, 118(3), 371-9. Absence of alpha-synuclein pathology in postencephalitic parkinsonism. Jellinger, K.A.], subacute sclerosing panencephalitis *[*Brain Dev., 2010, 32(6), 467-71. Tau proteins in the cerebrospinal fluid of patients with subacute sclerosing panencephalitis. Yuksel, D. *et al.*], amyotrophic lateral sclerosis/parkinsonism-dementia complex of Guam [Arch. Neurol., 1966, 15, 35—51. Amyotrophic lateral sclerosis and Parkinsonism-dementia complex on Guam. Further pathologic studies. Hirano, A. *et al.*], corticobasal degeneration [Psychol NeuroPsychiatr Vieil, 2009, 7(2), 91-100. Corticobasal degeneration: clinical and neuropsychological profile. Felician, O. *et al.*], pallido-ponto-nigral degeneration [Bull Acad Natl Med., 2000, 184(4), 799-809. Neurodegenerative disease associated with a mutation of codon 2 79 (N279K) in exon 10 of Tau protein. Delisle, M.B. *et al.*], prion disease [Biochem Soc Trans., 2010, 38(2), 545-51. Change in tau phosphorylation associated with neurodegeneration in the ME7 model of prion disease. Asuni, A.A. *et al.*], Gerstman-Straussler-Scheinker disease [Brain Pathol., 1995, 5, 61—75. Gerstmann—Sträussler—Scheinker disease and the Indiana kindred. Ghetti, B. *et al.],* Hallervordern—Spatz disease [J Neurol Sci., 2000, 177(1), 48-59. Widespread expression of alpha-synuclein and tau immunoreactivity in Hallervorden-Spatz syndrome with protracted clinical course. Saito, Y. *et al.*], myotonic dystrophy [Acta Neuropathol., 1991, 82, 1—5. Presenile appearance of abundant Alzheimer's neurofibrillary tangles without senile plaques in the brain in myotonic dystrophy. Kiuchi, A. *et al.*], squizophrenia [Prog Neuropsychopharmacol Biol Psychiatry., 2006, 30, 30(8), 1369-80. Dysregulation of tau phosphorylation is a hypothesized point of convergence in the pathogenesis of Alzheimer's disease, frontotemporal dementia and schizophrenia with therapeutic implications. Deutsch, S.I. *et al.*], multiple sclerosis *[*Acta Neuropathol., 2010, 119(5), 591-600. Abnormal tau phosphorylation in primary progressive multiple sclerosis. Anderson, J.M. *et al.*], dementia and normal aging [Acta Neuropathol., 1977, 37, 111—118. Neuronal loss, neurofibrillary tangles and granulovacuolar degeneration in the hippocampus with ageing and dementia. A quantitative study. Ball, M.J.*;* J. Neurol. Sci., 1968, 7, 331—356. Observations on the brains of non-demented old people. Tomlinson, B.E. *et al.*] and glaucoma [Neurobiol Aging., Available online 7 April 2009. Tau inclusions in retinal ganglion cells of human P301S tau transgenic mice: Effects on axonal viability. Gasparini, L. *et al.*].

For a review of pathologies wherein hyperphosphorylated tau protein is involved, please see Brain Research Reviews, 2000, 33, 95 —130. Tau protein isoforms, phosphorylation and role in neurodegenerative disorders. Buée, L. *et al.;* Eur J Neurol., 2009, 16(3), 297—309. Tauopathies with parkinsonism: clinical spectrum, neuropathologic basis, biological markers, and treatment options. Ludolph, A. C. *et al.*

In view of the above, it is of great potential therapeutic interest to find further CDK5, GSK-3 or CK1ε inhibitors, which may be useful for treating several diseases or conditions as detailed above.

**Indole-pyrimidine compounds**

A few indole-pyrimidine compounds have been described in the Art.

W02005/123672 discloses an extremely broad range of compounds, including, among many others, indole-pyrimidine derivatives, which are said to be inhibitors of a broad range of enzymes, particularly phosphoryl transferases, including kinases. Only a few particular compounds are disclosed, and experimental data are only included for one compound, specifically for A1K (Aurora A) and c-Kit kinases inhibitory activity.

Bioorganic & Medicinal Chemistry Letters (2011), 21(6), 1724-1727 describes compounds inhibiting TAK1, which is involved in pathways related to cell proliferation, tumor growth and inflammation. A few oxindole-aminopyrimidine compounds are descibed as synthesis intermediates, not displaying any biological activity.

**SUMMARY OF THE INVENTION**

A new family of compounds has been found, namely indole-pyrimidine derivatives which exhibit an improved ability to inhibit different kinases, such as Cdk5, GSK-3 and CK1ε with respect to other indole-pyrimidine derivatives, thus making these compounds better candidates for their therapeutic use in the treatment of pathologies related to these kinases.

Accordingly, in a first aspect, the present invention is related to a compound of Formula (I) :

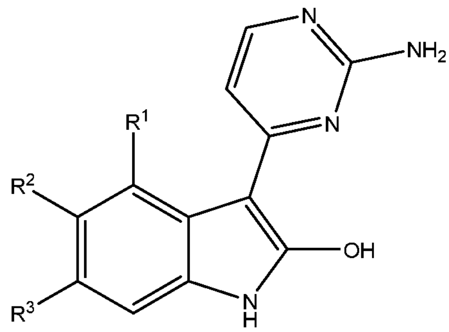

Formula I

wherein
$R^1$ is selected from hydrogen, halogen, OH, $NH_2$ and $NO_2$;
$R^2$ is selected from hydrogen, halogen, OH, $NH_2$ and- $NO_2$; and
$R^3$ is selected from hydrogen, halogen, $NH_2$, $NO_2$, -S $(O)_2$- $R^4$ and $COOR^4$, wherein $R^4$ is selected from hydrogen and alkyl;
or any pharmaceutically acceptable salt, solvate or prodrug thereof.

A second aspect of the present invention refers to a process (from now onwards process 1) for the preparation of a compound of Formula (I) which comprises:

a) protecting the amino reactive functional group of a compound of formula (II):

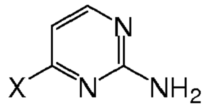

(II)

with a protecting group to form the compound of formula (III):

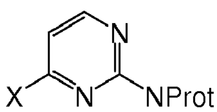

(III)

wherein X is a halogen selected from chloride and bromide and Prot is the protecting group of the amino group;
b) reacting the compound of formula (III) with an oxindole of formula (IV):

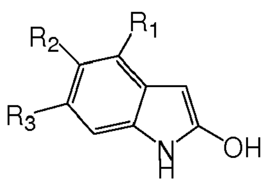

(IV)

wherein:

$R_1$, $R_2$ and $R_3$ are as defined above,

in the presence of a strong base, to form a compound of formula (V):

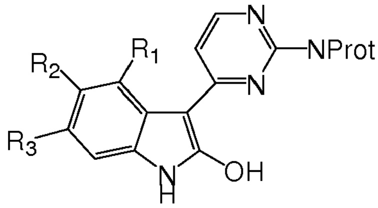

(V)

c) deprotecting the amino group of formula (V) to give the compound of formula (I).

An additional aspect of the present invention refers to a process (from now onwards process 2) for the preparation of a compound of Formula (I) which comprises:

a) reacting an oxindole of formula (IV):

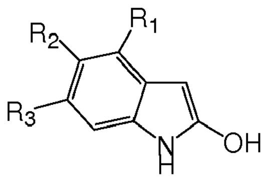

(IV)

with an electrophile of formula (VI):

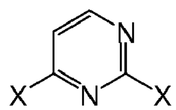

(VI)

to form a compound of formula (VII):

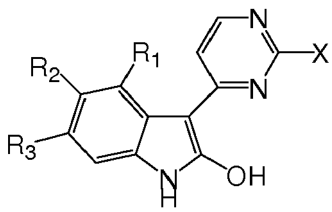

(VII)

and

b) reacting the compound of formula (VII) with ammonium hydroxide to form the compound of formula (I).

A further aspect of the present invention is a pharmaceutical composition comprising a compound of Formula (I) as defined above, or a salt, solvate or prodrug thereof, and at least one pharmaceutically acceptable carrier, adjuvant, and/or vehicle.

An additional aspect of the present invention refers to a compound of Formula (I), or a salt, solvate or prodrug thereof, for its use as a medicament.

Another aspect of the invention relates to a compound of Formula (I), or a salt, solvate or prodrug thereof, for its use in the treatment of a cognitive, neurodegenerative or neurological disease or condition.
A further aspect of the present invention refers to a compound of Formula (I), or a salt, solvate or prodrug thereof, for its use in the treatment of a disease or condition selected from diabetes, diabetes mellitus, inflammatory and autoimmune diseases, cardiovascular disorders, and pathologies selected from metabolic syndrome X, hair loss, severe acute respiratory syndrome coronavirus, stimulant drugs addiction such as cocaine addiction, bone loss and glaucoma.
An additional aspect of the present invention is the use of a compound of Formula (I) as defined above, or a salt, solvate or prodrug thereof, in the preparation of a medicament for the treatment of a cognitive, neurodegenerative or neurological disease or condition.
A further aspect of the present invention is the use of a compound of Formula (I) as defined above, or a salt, solvate or prodrug thereof, in the preparation of a medicament for the treatment of a disease or condition selected from diabetes, diabetes mellitus, inflammatory and autoimmune diseases, cardiovascular disorders, and pathologies selected from metabolic syndrome X, hair loss, severe acute respiratory syndrome coronavirus, stimulant drugs addiction such as cocaine addiction, bone loss and glaucoma.
Another aspect of the present invention is a method of treating a cognitive, neurodegenerative or neurological disease or condition, comprising administering to a patient in need of such treatment a therapeutically effective amount of a compound of Formula (I) as defined above, or a salt, solvate or prodrug thereof.
An additional aspect of the present invention is a method of treating a disease selected from diabetes, diabetes mellitus, inflammatory and autoimmune diseases, cardiovascular disorders, and pathologies selected from metabolic syndrome X, hair loss, severe acute respiratory syndrome coronavirus, stimulant drugs addiction such as cocaine addiction, bone loss and glaucoma, comprising administering to a patient in need of such treatment a therapeutically effective amount of a compound of Formula (I) as defined above, or a salt, solvate or prodrug thereof.
An additional aspect of the present invention is the use of a compound of Formula (I) as defined above, or a salt, solvate or prodrug thereof, as a reactive in an *in vitro* biological assay requiring inhibition of CDK5, GSK-3 or CK1ε.

## DETAILED DESCRIPTION OF THE INVENTION

### Definitions

In the present application the following terms have the meaning as indicated:

The term "alkyl" refers to a linear or branched hydrocarbon chain radical, said chain consisting of 1 to 6 carbon atoms, preferably, 1 to 3 carbon atoms, containing no insaturation, and which is attached to the rest of the molecule by a single bond, e. g., methyl, ethyl, n-propyl, i-propyl, n-butyl, t-butyl, n-pentyl, etc.

The term "halogen" refers to chloro, bromo, iodo or fluoro.
In a particular embodiment of the invention $R^1$ is hydrogen.
In another particular embodiment of the invention, $R^1$, $R^2$ and $R^3$ are hydrogen.
In another particular embodiment, $R^1$ and $R^3$ are hydrogen and $R^2$ is halogen or $NO_2$. More preferably, $R^1$ and $R^3$ are hydrogen and $R^2$ is halogen, even more preferably the halogen is chloro, bromo or fluoro.
In another particular embodiment, $R^1$ and $R^2$ are hydrogen and $R^3$ is $-S(O)_2-R^4$ or $-COOR^4$. In this particular embodiment, $R^4$ is preferably hydrogen or $C_1$-$C_6$ alkyl. More preferably $R^4$ is methyl.
In another particular embodiment of the invention, $R^1$ and $R^2$ are halogen and $R^3$ is hydrogen. More preferably, the halogen is fluoro.
Preferred compounds of the invention are selected from the following compounds:

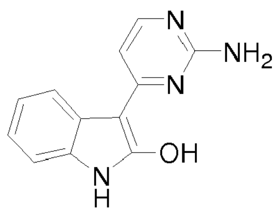

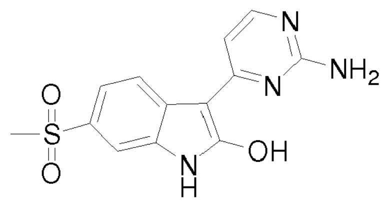

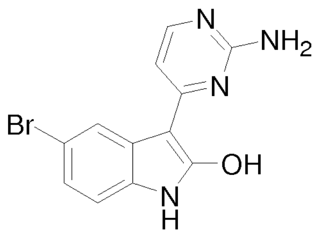

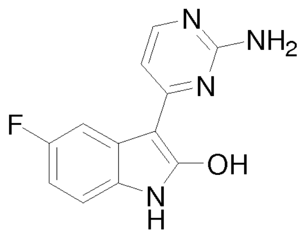

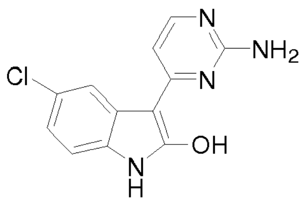

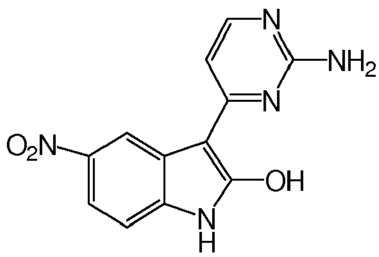

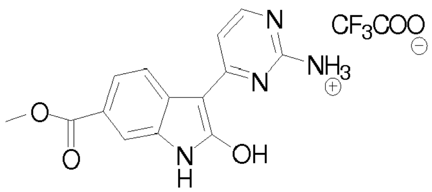

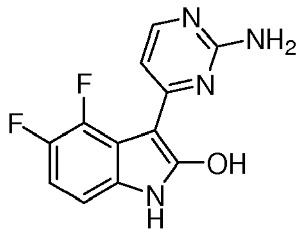

The term "pharmaceutically acceptable salts" refers to salts which, upon administration to the recipient are capable of providing (directly or indirectly) a compound as described herein. Further details are given below.

The term "prodrug" as used in this application is defined here as meaning a chemical compound having undergone a chemical derivation that yields the active compound per se after administration to a subject. Further details are given below.

The term "solvate" according to this invention is to be understood as meaning any form of the active compound according to the invention which has another molecule (most likely a polar solvent) attached to it via a non-covalent bonding.

**Synthesis of the compounds of the invention**

The compounds of formula (I) can be prepared following the process 1 depicted in scheme 1:

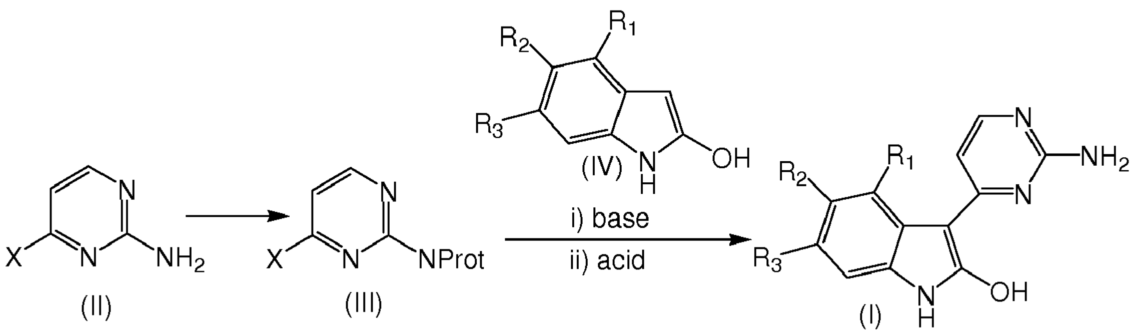


In process 1, it is necessary to protect the amino reactive functional group in order to avoid its unwanted participation in the subsequent reaction. Conventional protecting groups may be used in accordance with standard practice, for example see T.W. Greene and P.G.M. Wuts in "Protective Groups in Organic Chemistry", John Wiley and Sons, 1991. In a particular embodiment, the protective group is di- tert- butyl- dicarbonate.

The reaction of step b) is carried out in the presence of a strong base, such as NaH, KOH, LDA, etc. In particular,

the oxindole of formula (IV) is dissolved in an organic solvent, such as THF or DMSO, and the strong base is added to the solution as a previous step to the addition of the electrophile compound of formula (III). In a particular embodiment, the oxindole of formula (IV) is dissolved in DMSO and the strong base added in KOH.

In step c), the protecting group can be removed from the amino using acids, such as trifluoroacetic acid (TFA).

Alternatively, the compounds of formula (I) can be prepared by a process 2 depicted in scheme 2:

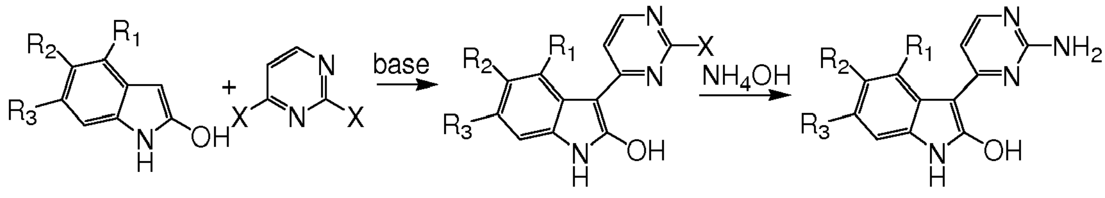


(IV) (VI) (VII) (I)

In process 2, the reaction of step a) is carried out in the presence of a strong base, such as NaH, KOH, LDA, etc. In particular, the oxindole of formula (IV) is dissolved in an organic solvent, such as THF or DMSO, and the strong base is added to the solution as a previous step to the addition of the electrophile compound of formula (VI) . In a particular embodiment, the oxindole of formula (IV) is dissolved in DMSO and the strong base added in KOH.

In another particular embodiment, the halogen groups in the compound of formula (VI) are chlorides.

Step b) can be carried out in a microwave reactor, and the mixture of the compound of formula (VII) and the ammonium hydroxide is subjected to different conditions of power, temperature and pressure depending on the desired product to be obtained.

It should also be appreciated that a variety of different solvents, temperatures and other reaction conditions can be varied to optimize the yields of the reactions.

In the experimental part, specific conditions to prepare the compounds of the invention according to processes 1) and 2) are disclosed.

In a particular embodiment, compound of formula:

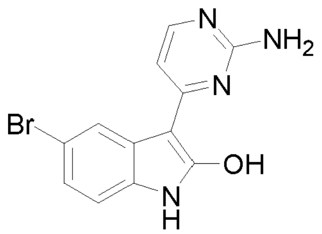

can also be isolated from *Aplidium sp* specie located around the Antarctic continent, as described in the experimental part of the present application.

**Medical Uses**

According to a preferred embodiment, the cognitive, neurodegenerative or neurological disease or condition in the above uses and methods of treatment is selected from Alzheimer's disease, Parkinson's disease, tauopathies degenerative dementias, dementias of mixed vascular and degenerative origin, senile dementia, frontotemporal dementia, familial frontotemporal dementia and parkinsonism linked to chromosome 17 due to mutations in the tau gene (FTDP-17- tau) and AIDS associated dementia; age- related diseases, Pick disease, progressive supranuclear palsy, subacute sclerosing panencephalitis, postencephalitic parkinsonism, dementia pugilistica, guam parkinsonism- dementia complex, corticobasal degeneration, argyrophilic grain disease, Huntington's disease, dementia with Lewy body disease (DLB), Lewy body variant of Alzheimer disease (LBVAD), Lewy body disease, bipolar disorder, depression, schizophrenia, epilepsy, electroconvulsive seizures, febrile seizures, mood disorders, anxiety and stress, circadian disorders, seasonal affective disorder, autism, attention deficit hyperactivity disorder, Down's syndrome, fragile X syndrome (FXS), ischemia/ reperfusion and diseases associated with ischemia/ reperfusion, shock, brain injury, traumatic brain injury, stroke, acute stroke, brain ischemia, acute neuronal injury, peripheral nerve injury, multiple sclerosis, autoimmune and inflammatory diseases afflicting the CNS, spinocerebellar ataxia type 1, cerebral bleeding such as cerebral bleeding due to solitary cerebral amyloid angiopathy, amyotrophic lateral sclerosis, mild cognitive impairment, amnestic mild cognitive impairment, Niemann- Pick disease, frontotemporal lobar degeneration, including progressive apraxia and progressive non-fluent aphasia, multiple system tauopathy with presenile dementia, chronic traumatic encephalopathy (CTE) and head trauma, pallido- ponto- nigral degeneration, prion disease, Gerstman- Sträussler- Scheinker disease, Hallervordern-Spatz disease, myotonic dystrophy, alcohol- induced neurodegeneration, fetal alcohol syndrome, diabetic neuropathy, focal cortical dysplasia, multiple system atrophy (MSA), pain, neuropathic pain, ataxia, hereditary canine spinal muscular atrophy and inclusion body myositis.

According to a preferred embodiment, the inflammatory and autoimmune disease or condition in the above uses and methods of treatment is selected from chronic inflammatory disease, rheumatoid arthritis, inflammatory bowel disease, psoriasis, arthritis, peritonitis, systemic inflammation, renal dysfunction , hepatotoxicity in endotoxemia, asthma, sepsis, colitis, inflammation-induced organ injury caused by hemorrhage and resuscitation, inflammatory injury in chronic renal allograft disease, and lupus.

According to a preferred embodiment, the cardiovascular disorders in the above uses and methods of treatment are selected from heart disease, atherosclerosis, hypertension, restenosis and leukopenia.

The term "pharmaceutically acceptable salts" refers to salts which, upon administration to the recipient are capable of providing (directly or indirectly) a compound as described herein. The preparation of salts can be carried out by methods known in the art. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p- toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N, N- dialkylenethanolamine, triethanolamine, glucamine and basic aminoacids salts.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., Textbook of Drug design and Discovery, Taylor & Francis (April 2002).

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "solvate" according to this invention is to be understood as meaning any form of the active compound according to the invention which has another molecule (most likely a polar solvent) attached to it via a non-covalent bonding. Examples of such solvates include hydrates and alcoholates, e.g. methanolates.

The preparation of salts, solvates and prodrugs can be carried out by methods known in the art. It will be appreciated that non-pharmaceutically acceptable salts, solvates or prodrugs also fall within the scope of the invention since those may be useful in the preparation of pharmaceutically acceptable salts, solvates or prodrugs.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention may exhibit tautomerism. Tautomers are one of two or more structural isomers of a compound that exist in equilibrium and are readily converted from one isomeric form to another. Common tautomeric pairs are amine-imine, amide-imidic acid, keto-enol, lactam-lactim, etc. For example, the compounds of the present invention may be equally represented as:

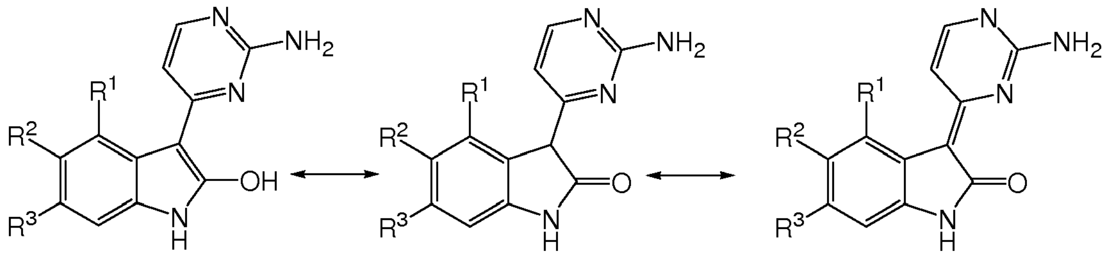

Unless otherwise stated, the compounds of the invention are also meant to include compounds which differ only in the presence of one or more isotopically enriched atoms. For example, compounds having the present structures except for the replacement of an hydrogen by a deuterium or tritium, or the replacement of a carbon by a $^{13}C$- or $^{14}C$-enriched carbon or $^{15}N$-enriched nitrogen are within the scope of this invention.

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

__Pharmaceutical compositions__

The present invention further provides pharmaceutical compositions comprising a compound of Formula (I) of the present invention, or a salt, solvate or prodrug thereof, and at least one pharmaceutically acceptable carrier, adjuvant, and/or vehicle, for administration to a patient.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions (*U.S. National Library of Medicine. National Institutes of Health*). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin.

According to a preferred embodiment, the pharmaceutical composition may further contain a therapeutically effective amount of one or more compounds useful for the treatment and/or prophylaxis of cognitive, neurodegenerative or neurological diseases or conditions. According to another embodiment, the pharmaceutical composition may further contain a therapeutically effective amount of one or more compounds selected from the group comprising beta secretase inhibitors or modulators including BACE1 protein inhibitors, amyloid beta- protein inhibitors, including immunoglobulins, antiamyloid monoclonal antibodies and vaccines, amyloid beta- protein precursor inhibitors, gamma secretase inhibitors or modulators, muscarinic receptor modulators, acetylcholinesterase inhibitors, butyrilcholinesterase inhibitors, Choline acetyltransferase stimulants, HMG- CoA reductase inhibitors, non- steroidal antiinflammatory agents, cyclo- oxygenase 2 inhibitors, N- methyl- D- aspartate receptor antagonists, vitamin E, nicotinic acetylcholine receptor modulators, serotonin receptor modulators, cannabinoid receptor agonists, CB1 receptor inverse agonists or CB1 receptor antagonists, AMPA receptor modulators, GABA receptor modulators, inhibitors of amyloid aggregation, glycogen synthase kinase beta inhibitors, promoters of alpha secretase activity, phosphodiesterase 9A and 10 inhibitors, type 4 cyclic nucleotide phosphodiesterase inhibitors, estrogen and cholesterol absorption inhibitors, 11- beta hydroxysteroid dehydrogenase type 1 inhibitors, adenosine receptor antagonists, adrenergic receptor modulators, advanced glycosylation end- product receptor antagonists, alpha- synuclein inhibitors, antioxidants, free radical scavengers, apolipoprotein A stimulants,

apolipoprotein E agonists, apoptosis inhibitors, calcium channel modulators, sodium channel modulators, calpain inhibitors, cathepsin B inhibitors, cell- replacements including stem- cell- therapies, glial cell line- derived neurotrophic factor agonists, nerve growth factor stimulants, chelating agents, complement factor D inhibitors, cyclic AMP response element-binding protein stimulants, D amino acid oxidase inhibitors, dopamine receptor agonists and dopamine uptake inhibitors, endopeptidase inhibitors, fibroblast growth factor stimulants, G protein- coupled receptor antagonists, gene expression stimulants, glucose stimulants, metabotropic glutamate receptor modulators, histamine H3 receptor antagonists or inverse agonists, histone deacetylase inhibitors, mitochondrial- permeability- transition pore- modulators, monoamine oxidase B inhibitors, neuropeptide stimulants, neurotransmitter modulators, plasminogen activator inhibitor- 1 inhibitors, protein kinase C stimulants, rho- associated kinase inhibitors, ribonucleotide reductase inhibitors, signal transduction pathway inhibitors, superoxide dismutase stimulants, tau protein modulators, tubulin polymerisation promoters, toll- like receptor agonists, transglutaminase inhibitors and Wnt protein modulatorsExamples of pharmaceutical compositions include any solid (tablets, pills, capsules, granules etc.) or liquid (solutions, suspensions or emulsions) composition for oral, topical or parenteral administration, among others.

Pharmaceutical dosage forms include but are not limited to parenteral preparations (such as injections, powders for injections, implants, etc), liquid preparations for oral use (such us syrups, solutions, suspensions, emulsions, powders and granules for suspension and for solution, oral drops, etc), oromucosal preparations (such as lozenges, sublingual and buccal tablets, oromucosal drops and sprays, etc) solid preparations for oral use (oral powders, effervescent powders, tablets- uncoated, coated, effervescent, soluble, dispersible, orodispersible, modified release, gastro- resistant, -oral lyophilisates, capsules — hard, soft, modified release, gastro- resistant-, granules — coated, effervescent, modified release, gastro- resistant- ), transdermal patches, powders for inhalation, nasal preparations and rectal preparations.

In a preferred embodiment the pharmaceutical compositions are in oral form because of the convenience for the patient and the chronic character of many of the diseases to be treated. Said oral pharmaceutical compositions may contain conventional excipients known in the art, such as:

<u>Film coated tablet:</u>

- Binders, such as maize starch, pregelatinised maize starch, povidone, gelatine, etc
- Diluents or fillers, such as microcrystalline cellulose, lactose, sodium phosphate, calcium phosphate dibasic dihydrate, calcium phosphate dibasic anhydrous (Emcompress, Di- tab, Di- cal- fos), etc
- Disintegrants, such as sodium croscarmellose (Acdisol, Explocel, Vivasol), sodium starch glycolate (Glycolis, Explotab, Primojel, Vivastar), cross-linked povidone, gums, etc.
- Glidants, such as talc or colloidal silica.
- Lubricants, such as magnesium stearate, stearic acid, sodium stearyl fumarate, etc
- Film-formers, such as hydroxypropylcellulose (Klucel, Metocel), Hypromellose (Metocel, Metolose, Pharmacoat), hydroxypropylmethylcellulose, etc
- Opacifiers, such as titanium dioxide.
- Colouring agents, such as sunset yellow, iron oxides, indigo carmine, erythrosine, etc
- Plasticizers, such as polyethyleneglycol, triacetin, etc

<u>Powder for oral solution (POS) in sachet</u>

- Acidifying agents, such as citric acid.
- Buffering agents, such as citric acid, sodium citrate
- Diluents or fillers, such as mannitol (Pearlitol), sorbitol (Neosorb, Parteck), sucrose, maltose (Advantose), etc

- Sweetening agents, such as sucralose, aspartame, accesulfame, sodium saccharine, etc
- Glidants, such as colloidal silicon dioxide (Aerosil, Cabosil, Aeroperl)
- Flavouring agents, such as strawberry flavour, lemon flavour, cola flavour, orange flavour, etc
- Thickening or stabilisers such as modified celluloses (hydroxipropylcellulose, carboxymethylcellulose sodium, ...), povidones, gums, etc

Syrup

- Antimicrobial and solvent agents, such as ethanol, propyleneglycol, etc.
- Sweetening agents, such as sorbitol or sucrose
- Antimicrobial preservative, such as sodium benzoate, potassium sorbate
- Acidifying agents, such as citric acid or ascorbic acid
- Buffering agents, such as citric acid and sodium citrate, phosphates, acetic acid and sodium acetate.
- Flavouring agents, such as vainille flavour, strawberry flavour, cola flavour, peach flavour, etc
- Colouring agents, such as tartrazine, curcumin, quinoline yellow, sunset yellow, etc

Capsules

- Diluents, such as microcrystalline cellulose, lactose, calcium carbonate, calcium phosphate dibasic, calcium phosphate monobasic, calcium sulphate
- Disintegrants, such as sodium starch glycolate, cross-linked povidone.
- Lubricants, such as talc, magnesium stearate, stearic acid, sodium stearyl fumarate, polyethylenglycols, etc.

Gastrorresistant capsules

- Capsule fillers, such as microcrystalline cellulose, sugar spheres.
- Binders and film formers, such as copolymers methacrylate acid, polymeric methacrylates (Eudragit, Kollicoat)
- Plasticizers and film formers such as dibutylphthalate
- Colouring agents, such as erythrosine, sunset yellow, indigo carmine, etc
- Solvents, such as acetone, isopropyl alcohol, etc

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating. Particular examples are given below.

The pharmaceutical compositions may also be adapted for parenteral administration, such as sterile solutions,

suspensions or lyophilized products in the appropriate unit dosage form. Adequate excipients can be used, such as:

- Antimicrobial preservatives, such as methylparaben, prophylparaben, etc.
- Antioxidants, such as sodium metabisulfite, propyl gallate, etc
- Stabilizing and suspending agents, such as soluble or swellable modified celluloses, e.g. carboxymethylcellulose sodium (Aquasorb, Blanose, Nymcel)
- Tonicity agents, such as sodium chloride
- Solubilizers, such as propyleneglycol or polyethyleneglycols

Particular examples are given below.

The mentioned formulations will be prepared using standard methods such as those described or referred to in the Spanish and US Pharmacopoeias and similar reference texts.

In the following, the present invention is further illustrated by examples. They should in no case be interpreted as a limitation of the scope of the invention as defined in the claims.

## EXAMPLES

### PREPARATION OF THE COMPOUNDS

**Example 1. Method of synthesis of N-Boc-chloropyrimidine (Intermediate 1)**

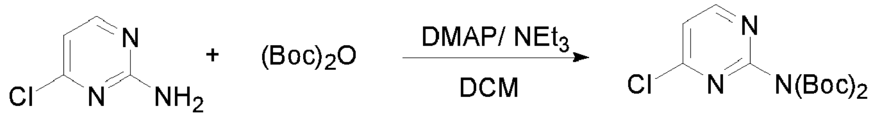


**Intermediate 1**

A mixture of 2- amino- 4- chloro- pyrimidine (7, 5 mmol, 1g) with DMAP (0, 2 eq, 1, 5 mmol, 183, 5 mg), triethylamine (0, 2 eq, 1, 5 mmol, 0, 2 ml) and di- tert- butyl- dicarbonate (2 eq, 15 mmol, 3, 2 g) in dichloromethane (60 mL) was stirred at room temperature for 48 h. When the reaction was finished the solvent is evaporated and the resulting crude was dissolved in $CH_2Cl_2$ and washed with water. The organic phase was evaporated to dryness in vacuo and the residue purified by silica gel column chromatography using as eluent AcOEt/ Heptane in gradient.
NMR ($CDCl_3$) : $\delta$ ppm 7.9 (d, 1H) ; 6.52 (d, 1H) ; 1.43 (s, 18 H)

**Example 2. Methods of preparation of End products (General procedures)**

There are two general methods to prepare 2-oxoindole derivatives:

**Method A:**

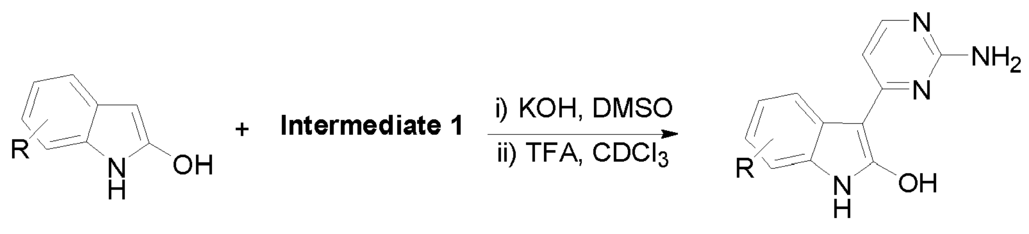


6-R= COOMe

The oxindole in DMSO is treated with KOH (1,2 eq) at room temperature and allowed to stir for 1 h. The electrophile (1eq) 1 is added and the reaction is stirred at room temperature overnight. When the reaction is finished $H_2O$ is added and treated with HCl 1M until pH=1. Then the aqueous phase is extracted with AcOEt (x2). The organic phase was dried with $MgSO_4$ anhydrous and evaporated in vacuo. The residue dissolved in $CDCl_3$ is treated with TFA in exceed at room temperature overnight. Then, $H_2O$ is added and the product appeared as a fine precipitate that after be filtered it is not necessary any purification.

<u>**Method B:**</u>

**Step 1:**

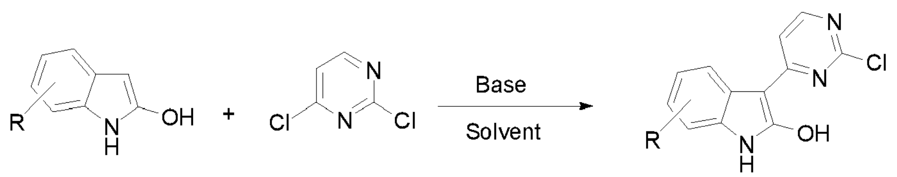


**Step 2:**

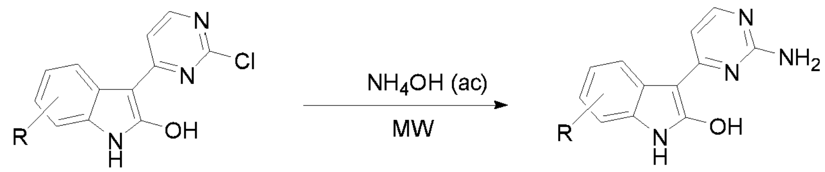


1. The oxindole dissolved in the corresponding solvent (THF, DMF, DMSO...etc) is treated with the appropriate strong base (NaH, KOH, LDA... etc) and allowed to stir for 0.5 to 1 h. The temperature can range between -20°C to room temperature. Then, the electrophile is added and the reaction is finished after 18-24 h at room temperature. The residue is treated with $H_2O$ and organic solvent (DCM or AcOEt). The aqueous phase is washed with more organic solvent (x2) and treated with HCl 1N to obtain practically pure the intermediate product as fine precipitate.

2. To transform the chloro-pyrimidine in amino-pyrimidine, the mixture of the intermediate product in ammonium hydroxide solution 32 % in the microwave reactor is subjected to the next conditions in the CEM Discover Microwave:

Power: 150 W; Ramp time: 5 min.; Hold time: 250 min.; Temperature: 110 °C; Press: 150 psi.

The reaction is evaporated to dryness and the product is purified by, for example, preparative/ semipreparative HPLC or a simple washed with $CH_2Cl_2$/MeOH depending on the case.
All compounds were detected on a Waters ZQ LC/MS single cuadrupole system (Column Desc Sunfire C18 3.5 $\mu$m 2.1x100mm. Gradient FMA/FMB (0.1 % Formic acid in $H_2O$/ 0.1 % Formic acid in AcN))

Following the above general procedures, the following compounds according to the invention were prepared:

**Table 1**

| Compound No. | Structure |
|---|---|
| Compound 1 | 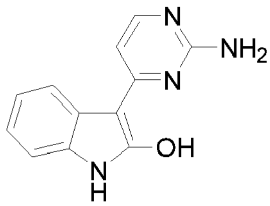 |
| Compound 2 | 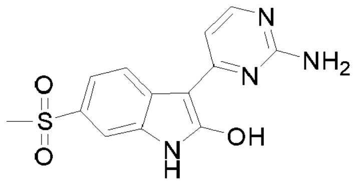 |
| Compound 3 | 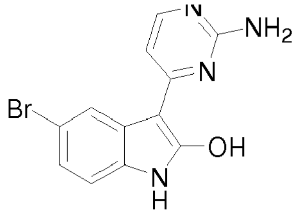 |
| Compound 4 | 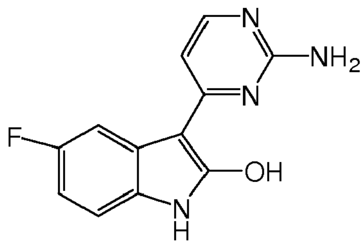 |
| Compound 5 | 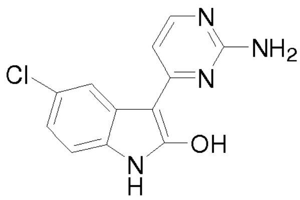 |
| Compound 6 | 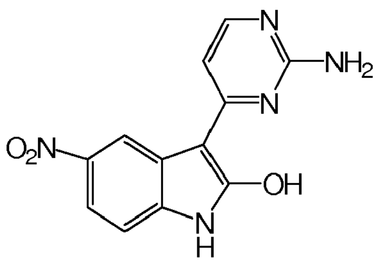 |
| Compound 7 | 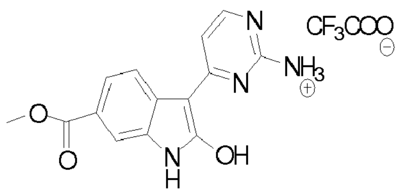 |

(continued)

| Compound No. | Structure |
| --- | --- |
| Compound 8 | 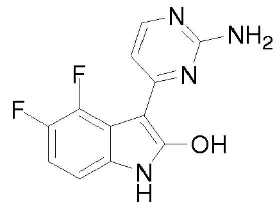 |

In the following, the particular reagents necessary for obtaining the above compounds, as well as their experimental spectral data, are indicated.

**Example 3. Isolation and identification of 3-(2-Amino-4-pyrimidinyl)-5-Bromo-2-oxoindole (Compound 3)**

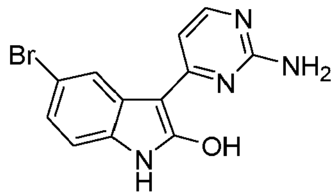

The pale brown tunicate *Aplidium sp* was collected around the Antarctic continent. A voucher specimen is deposited at PharmaMar in Colmenar Viejo (Spain) .
The frozen tunicate was chopped and lyophilised overnight (18 g.), the sample was triturated and extracted three times with dichloromethane/ methanol 1: 1, the combined extracts was taken to dryness under reduced pressure to yield 1.80 g. of a yellow organic crude. In other way, the residue was extracted with 50 ml of water to generated 543 mg of an aqueous extract.
The organic extract was subjected to flash- chromatography on reversed phase silica with the use of a $H_2O$—MeOH gradient, affording four fractions. Fraction three (124 mg) was purificated by preparative high performance liquid chromatography (HPLC) (Sunfire C- 18 21x250mm, gradient H2O/ ACN+0.1%AF from 5 to 100% ACN in 30 min, UV detection at 254nm) to achieve different fractions, one of them was a yellow pure **compound 3** (microgrames) .
It was not possible to obtain an optimal $^1H$, $^{13}C$, NMR spectra due to the very low quantity of isolated compound. Mass spectrometry was performed and the results were compared with other known compounds to elucidate the chemical structure of compound 3. Subsequently, this compound was synthesised and the UV, mass spectrometry and biological activity of both the natural and the synthetic product were compared and confirmed to be the same compound.

**Example 4. Synthesis of 3-(2-Amino-4-pyrimidinyl)-5-Bromo-2-oxoindole (Compound 3)**

Method B. Reagents (step 1) : 5- Bromo- 2- Oxoindole (2.3 mmol, 500 mg) ; 2, 4- Dichloropyrimidine (2.6 mmol, 386 mg) ; NaH 60 % in mineral oil (5.9 mmol, 236 mg) ; Solvent: DMF (5 ml)
Reagents (step 2) : 3- (2- Chloro- 4- pyrimidinyl)- 5- Bromo- 2- oxoindole (3.1 mmol, 100 mg) ; $NH_4OH$ 32% (7.5 mL)
Purification: washed with $CH_2Cl_2$/ MeOH
$^1H$ NMR (DMSO- d6), δ ppm 7.07 (s, 1H, *indol*) , 6.93 (d, *J* = 7.80 Hz, 1H, *pyrimidine),* 6.84 (d, *J* = 8.37 Hz, 1H, *indol*) , 6.52 (d, *J* = 8.39 Hz, 1H, *indol*) , 6.30 (d, *J* = 7.93 Hz, 1H, *pyrimidine) .*
$^{13}C$NMR (DMSO- $d_6$) : 170.0, 157.9, 154.7, 151.4, 132.9, 132.8, 109.646, 109.4, 104.9, 102.0, 101.9, 85.9.
ESI- MS: m/z 305.9 $(M+H)^+$, 100%, 307.9 $(M+2H)^+$, 98%.

**Example 5. 3-(2-Amino-4-pyrimidinyl)-2-oxoindole (Compound 1)**

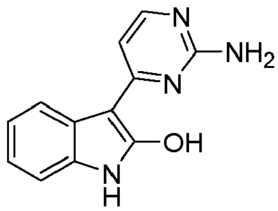

Method B. Reagents (step 1) : 2- Oxoindole (15 mmol, 2 g) ; 2, 4- Dichloro- pyrimidine (22.5 mmol, 3.35 g) ; KOH (30 mmol, 1.68 g) ; Solvent: DMSO anhydrous (10 ml)
Reagents (step 2) : 3- (2- Chloro- 4- pyrimidinyl)- 2- oxoindole (2 mmol, 512.5 mg) ; $NH_4OH$ 32% (7 mL)
Purification: washed with $CH_2Cl_2$/ MeOH
$^1H$- NMR (DMSO- $d_6$) : 13.7 (1H, br, NH) ; 10.4 (1H, br, NH) ; 7.8 (d, 1H, *Hpyrimidine) ;* 7.4 (m, 1H, *Hindol) ;* 6.8- 6.9 (m, 3H, *Hpyrimidine) ;* 6.6 (d, 1H, H*pyrimidine*)
$^{13}C$- NMR (DMSO- $d_6$) : 169.4; 163.0; 156.0; 153.8; 149.1; 135.0; 124.4; 121.1; 119.9; 117.3; 108.6; 100.0
ESI- MS: m/z 227 $(M+H)^+$ 100%

**Example 6. 3-(2-Amino-4-pyrimidinyl) -6-methanesulfonyl -2-oxoindole (Compound 2)**

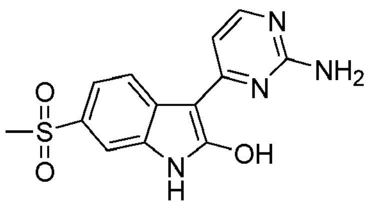

Method B. Reagents (step 1) : 6- Methanesulfonyl- 2- Oxoindole (1.4 mmol, 300 mg) ; 2, 4- Dichloro- pyrimidine (1.8 mmol, 209 mg) ; KOH (2.8 mmol, 157mg) ; Solvent: DMSO anhydrous (3 ml)
Reagents (step 2) : 3- (2- Chloro- 4- pyrimidinyl)- 6- methanesulfonyl- 2- oxoindole (0.93 mmol, 301 mg) ; $NH_4OH$ 32% (7 mL)
Purification: Preparative HPLC on Waters preparative Delta 600 with DAD 2996. Column: C18- Sun Fire 19x250 mm. Flow: 17.5 ml/min. Mobil Phase: A, 0.1 % Formic acid in $H_2O$; B, 0.1 % Formic acid in AcN) . Gradient:

| Time/min. | %A | %B |
|---|---|---|
| 0 | 95 | 5 |
| 5 | 80 | 20 |
| 35 | 0 | 100 |
| 40 | 0 | 100 |
| 45 | 95 | 5 |
| 55 | 95 | 5 |

ESI-MS: m/z 305.0 $(M+H)^+$ 100%

**Example 7. 3-(2-Amino-4-pyrimidinyl) -5-Fluoro-2-oxoindole (Compound 4)**

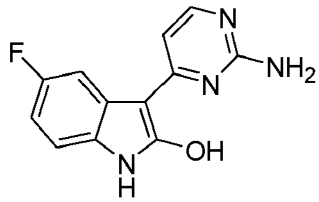

Method B. Reagents (step 1) : 5- Fluoro- 2- Oxoindole (1.32 mmol, 200 mg) ; 2, 4- Dichloro- pyrimidine (1.5 mmol, 223 mg) ; KOH (2.64 mmol, 148 mg) ; Solvent: DMSO anhydrous (3ml)
Reagents (step 2) : $NH_4OH$ 32% (7 mL)
Purification: Preparative HPLC on Waters preparative Delta 600 with DAD 2996. Column: C18- Sun Fire 19x250 mm. Flow: 17.5 ml/min. Mobil Phase: A, 0.1 % Formic acid in $H_2O$; B, 0.1 % Formic acid in AcN) . Gradient:

| Time/min. | %A | %B |
|---|---|---|
| 0 | 95 | 5 |
| 5 | 50 | 50 |
| 30 | 30 | 70 |
| 36 | 0 | 100 |
| 40 | 0 | 100 |
| 45 | 95 | 5 |
| 55 | 95 | 5 |

ESI-MS: m/z 245.1 $(M+H)^+$ 100%

**Example 8. 3-(2-Amino-4-pyrimidinyl) -5-Chloro -2-oxoindole (Compound 5)**

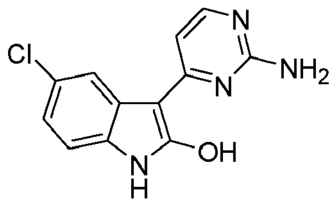

Method B. Reagents (step 1) : 5- Chloro- 2- Oxoindole (1.8 mmol, 300 mg) ; 2, 4- Dichloropyrimidine (1.8 mmol, 268 mg) ; KOH (3.6 mmol, 202 mg) ; Solvent: DMSO anhydrous (3 ml)
Reagents (step 2) : 3- (2- Chloro- 4- pyrimidinyl)- 5- Chloro- 2- oxoindole (0.89 mmol, 250 mg) ; $NH_4OH$ 32% (7 mL)
Purification: Preparative HPLC on Waters preparative Delta 600 with DAD 2996. Column: C18- Sun Fire 19x250 mm. Flow: 17.5 ml/min. Mobil Phase: A, 0.1 % Formic acid in $H_2O$; B, 0.1 % Formic acid in AcN) . Gradient:

| Time/min. | %A | %B |
|---|---|---|
| 0 | 95 | 5 |
| 5 | 70 | 30 |
| 35 | 0 | 100 |
| 40 | 0 | 100 |
| 45 | 95 | 5 |
| 55 | 95 | 5 |

ESI-MS: m/z 260.8 $(M+H)^+$ 100%; 262.8 $(M+2H)^+$ 32%

**Example 9. 3-(2-Amino-4-pyrimidinyl) -5-Nitro -2-oxoindole (Compound 6)**

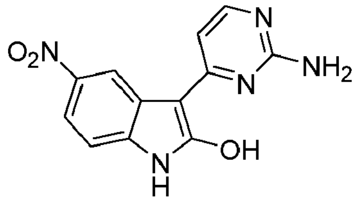

Method B. Reagents (step 1) : 5- Nitro- 2- Oxoindole (1.68 mmol, 300 mg) ; 2, 4- Dichloropyrimidine (2 mmol, 297.5 mg) ; KOH (3.36 mmol, 188 mg) ; Solvent: DMSO anhydrous (3ml)
Reagents (step 2) : 3- (2- Chloro- 4- pyrimidinyl)- 5- Nitro- 2- oxoindole (0.65 mmol, 189 mg) ; $NH_4OH$ 32% (7 mL)
Purification: Preparative HPLC on Waters preparative Delta 600 with DAD 2996. Column: C18- Sun Fire 19x250 mm. Flow: 17.5 ml/min. Mobil Phase: A, 0.1 % Formic acid in $H_2O$; B, 0.1 % Formic acid in AcN) . Gradient:

| Time/min. | %A | %B |
|---|---|---|
| 0 | 80 | 20 |
| 5 | 80 | 20 |
| 20 | 0 | 100 |
| 25 | 0 | 100 |
| 26 | 80 | 20 |
| 35 | 80 | 20 |

ESI-MS: m/z 272.0 $(M+H)^+$ 100%

**Example 10. 3-(2-Amino-4-pyrimidinyl) -2-oxoindole-6-carboxylic acid methyl ester (Compound 7)**

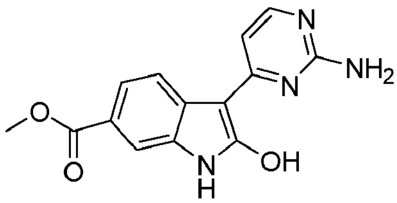

Method A. Reagents: 2- Oxoindole- 6- carboxylic acid methyl ester (1.05 mmol, 200 mg) ; 2- N- (Boc)- 4- Chloro-pyrimidine (1.2 mmol, 395 mg) ; KOH (2 mmol, 118 mg) ; DMSO anhydrous (2.5 ml) ; TFA (exceed)
ESI- MS: m/z 285.1 $(M+H)^+$ 100 %

**Example 11. 3-(2-Amino-4-pyrimidinyl)-4,5-difluoro-2-oxoindol (Compound 8)**

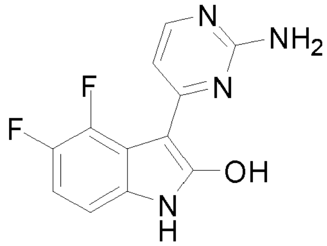

Method B. Reagents (Step 1) : 4, 5- difluoro- 2- oxoindol (1.8 mmol, 300.5 mg), 2, 4 dichloropyrimidine (2.7 mmol, 402 mg), KOH (3.6 mmol, 209.4 mg) . Solvent: DMSO anhydrous (5 mL) .
Reagents (Step 2) : $NH_4OH$ 32 % (117.1 mmol, 7 mL) .
Purification: Silica gel column chromatography (eluent: DCM/$NH_3$ in MeOH 3.5N from 0% to 100%) . After evaporation of the solvent the resulting solid was washed with $Et_2O$.
$^1$H- NMR (DMSO- $d_6$), $\delta$ ppm 10.74 (s, 1H, NH *indol),* 7.90 (d, *J* = 6.18 Hz, 1H, *pyrimidine),* 7.79 (s, 2H, $NH_2$), 6.96- 6.85 (m, 1H, *indol),* 6.82- 6.77 (m, IH *pyrimidine),* 6.67- 6.62 (m, 1H, *indol*) .
$^{13}$C- NMR (DMSO- $d_6$) : 170.0, 157.9, 154.7, 151.5, 132.9, 132.8, 109.6, 109.5, 104.9, 102.1, 101.9, 85.9.
ESI- MS: m/z 263.0 $(M+H)^+$, 100%

## BIOLOGICAL DATA

### Example 12: Cdk5 assay

The enzymatic activity of Cdk5 is determined with a commercial system, available from Promega (Madison, WI, USA) based on the luminescent detection of ADP, the byproduct of the kinase reaction. Human recombinant Cdk5/p35 heterodimer (purchased from Life Technologies, Carlsbad, CA, USA) is used as the enzyme source. At the end of the enzymatic reaction the remaining ATP is destroyed and afterwards the ADP produced during the kinase reaction is converted into ATP, which is further converted into light using a suitable luciferase. Therefore, the luminescent signal positively correlates with kinase activity. The assay is performed in 96-well conical bottom black plates, in a final volume of 12 $\mu$l with 10 nM enzyme concentration in 40 mM Tris-HCl pH 7.4, 20 mM $MgCl_2$ and 0.01% bovine serum albumin using 10 $\mu$M ATP and 2 $\mu$M substrate. The latter is histone H1, a 219 amino acids-size protein (SwissProt: P10412) purified from calf thymus and provided by Millipore (Billerica, MA, USA). The assay is carried out in the presence of different concentrations of the tested compound, at a final 1% (v/v) DMSO concentration. After a 40 min incubation, at room temperature, 12 $\mu$l of a commercial "ADP Glo" solution (from Promega) is added to remove the remaining ATP and a subsequent 40 min incubation at room temperature is performed before adding 24 $\mu$l of a suitable "detection reagent" (also supplied by Promega) needed to carry out the transformation of ADP into ATP and its conversion into light by the luciferase enzyme included in the reagent. After a final 30 min incubation at room temperature luminescence is monitored in a suitable plate reader. In the assay plate several wells are included as a control for full kinase activity, these wells do not contain any inhibitor or tested substance. Likewise, several wells are also included as a control for the lack of kinase activity, these wells do not contain inhibitor nor Cdk5/p35. The luminescence signal yielded by each tested sample is normalized to that of the control wells, so that for every compound concentration the percentage of inhibition is calculated by using the following equation:

$$\%\,\text{Inhibition} = 100 \cdot \frac{E-S}{E-B}$$

where "S" is the luminescence of the wells containing the tested sample, "E" is the average luminescence of the wells including Cdk5/p35 in the absence of inhibitors, and "B" is the average luminescence of the wells without kinase. The inhibition values obtained at every compound concentration are finally used to calculate the pEC50 of the tested compound, this parameter being the negative logarithm of the compound concentration which causes 50% of inhibition. For that purpose the data were fitted to a version of the classical 4-parameters isotherm equation using the nonlinear regression function of GraphPad™ Prism 5.0 (GraphPad Software Inc.). Such equation is described below:

$$\% \text{ Inhibition} = L + \frac{H - L}{1 + 10^{(\log C \, + \, pEC50)\, n}}$$

where "L" is the lower asymptote of the theoretical sigmoidal curve, "H" is the higher asymptote, "C" is the concentration of compound and "n" is the Hill coefficient.

In **Table** 2, the pEC50 values obtained for some compounds of Formula I are indicated:

**Table 2**

| Compound No | Structure | Cdk5 inhibition pEC50 average |
|---|---|---|
| Compound 1 | See table 1 | 8.2 |
| Compound 2 | See table 1 | 6.7 |
| Compound 3 | See table 1 | 8.3 |
| Compound 4 | See table 1 | 7.5 |
| Compound 5 | See table 1 | 8.8 |
| Compound 6 | See table 1 | 6.9 |
| Compound 7 | See table 1 | 6.8 |
| Compound 8 | See table 1 | 7.6 |
| Comparative example | 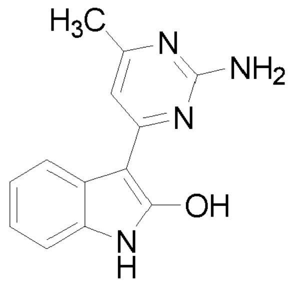 | 6.3 |

**Example 13: GSK-3β assay**

The enzymatic activity of GSK- 3β is determined with the same commercial system described above based on the luminescent detection of ADP. Human recombinant GSK- 3β (purchased from Millipore) is used as the enzyme source. The assay is performed in 96- well conical bottom black plates, in a final volume of 12 μl with 4 nM enzyme concentration in 40 mM Tris- HCl pH 7.4, 20 mM $MgCl_2$ and 0.01% bovine serum albumin using 5 μM ATP and 15 μM substrate peptide. The latter is a synthetic peptide supplied by Millipore under the commercial name "Phospho- Glycogen Synthase Peptide- 2", and it is based upon the sequence of skeletal muscle glycogen synthase including sites 3b, 3c and phosphorylated site 4 from this protein (the exact sequence of the peptide is YRRAAVPPSPSLSRHSSPHQ (pS) EDEEE, where (pS) denotes phospho- Ser) . The assay is carried out in the presence of different concentrations of the tested compound at a final 1% (v/v) DMSO concentration. After a 40 min incubation at room temperature samples are processed as described above and the luminescence signal yielded by each tested sample is used to calculate the pEC50 value as described above for Cdk- 5.

In **Table 3,** the pEC50 values obtained for some compounds of Formula I are indicated:

**Table 3**

| Compound No | Structure | GSK3β inhibition pEC50 average |
|---|---|---|
| Compound 1 | See table 1 | **6.9** |
| Compound 2 | See table 1 | **5.3** |

(continued)

| Compound No | Structure | GSK3β inhibition |
|---|---|---|
| | | pEC50 average |
| Compound 3 | See table 1 | **7.8** |
| Compound 4 | See table 1 | **7.5** |
| Compound 5 | See table 1 | **7.9** |
| Compound 6 | See table 1 | **6.7** |
| Compound 7 | See table 1 | **6.6** |
| Compound 8 | See table 1 | **7.6** |
| Comparative example | See table 2 | **6.4** |

**Example 14: CK1ε assay**

The enzymatic activity of CK1ε is determined with the same commercial system described above based on the luminescent detection of ADP. Human recombinant CK1ε (purchased from from Life Technologies) is used as the enzyme source. The assay is performed in 96- well conical bottom black plates, in a final volume of 12 μl with 10 nM enzyme concentration in 40 mM Tris- HCl pH 7.4, 20 mM $MgCl_2$ and 0.01% bovine serum albumin using 50 μM ATP and 50 μM substrate peptide. The latter is a synthetic peptide provided by AnaSpec (Fremont, CA, USA) under the commercial name "CK1 peptide substrate" and it is based on the sequence of rabbit muscle glycogen synthase with Ser7 phosphorylated (the exact sequence of the peptide is KRRRAL (pS) VASLPGL, where (pS) denotes phospho- Ser) . The assay is carried out in the presence of different concentrations of the tested compound at a final 1% (v/v) DMSO concentration. After a 40 min incubation at room temperature samples are processed as described above and the luminescence signal yielded by each tested sample is used to calculate the pEC50 value as described above for Cdk- 5.

In **Table 4,** the pEC50 values obtained for some compounds of Formula I are indicated:

**Table 4**

| Compound No | Structure | CK1 inhibition |
|---|---|---|
| | | pEC50 average |
| Compound 1 | See table 1 | 6.1 |
| Compound 2 | See table 1 | 5.4 |
| Compound 3 | See table 1 | 6.1 |
| Compound 4 | See table 1 | 6.5 |
| Compound 5 | See table 1 | 6.7 |
| Compound 6 | See table 1 | 4.7 |
| Compound 7 | See table 1 | 5.9 |
| Compound 8 | See table 1 | < 4 |
| Comparative example | See table 2 | 5.5 |

**Example 15: Cellular assay**

The effect of the compounds in cells is determined by monitoring the level of phosphorylation at the Ser-396 residue of Tau protein in human neuroblastoma SH-SY5Y cells. Cells are grown in 96-well plates, 30,000 cells per well in a volume of 100 μl of 1:1 MEM:Ham's F12 medium including 10% (v/v) fetal bovine serum, 100 U/ml penicillin, 100 μg/ml streptomycin and 1% (v/v) 100-fold concentrated commercial stock of non-essential aminoacids (Gibco, Carlsbad, CA, USA), and incubated at 37°C during 24 h. Then, the corresponding amount of test compound is added to the wells to reach the desired concentration in 1% (v/v) DMSO. The system is incubated at 37°C during 16 h and afterwards cells are harvested, washed with phosphate buffer saline and incubated during 30 min on ice with 120 μl per well of "lysis buffer" (10 mM Tris-HCl pH 7.4, 100 mM NaCl, 1 mM EDTA, 2 mM sodium orthovanadate, 1% (v/v) Triton X-100, 10%

(v/v) glycerol, 0.1% (w/v) sodium dodecyl sulfate, 0.5 % (w/v) sodium deoxycholate, 4% (v/v) 25-fold concentrated commercial stock of protease inhibitors (Roche, Basel, Switzerland) and 1 mM phenylmethylsulfonyl fluoride). 60 μl of this mixture is then placed in an empty 96-well plate and the levels of Ser-396 phosphorylation are determined using a commercial ELISA kit (Biosource, Carlsbad, CA, USA) following manufacturer's instructions. The levels of phosphrylation are expressed as percentage values after normalizing them to those in untreated cells and those values are used to calculate the pEC50s as described above.

In **Table 5,** the pEC50 values obtained for some compounds of Formula I are indicated:

**Table 5**

| **Compound No** | **Structure** | **pSer396-Tau 16h** |
|---|---|---|
| | | **pEC50 average** |
| Compound 4 | See table 1 | 5.9 |
| Compound 5 | See table 1 | 6.5 |
| Compound 6 | See table 1 | 5.0 |

**PHARMACEUTICAL COMPOSITIONS**

In the following, the detailed preparation of some pharmaceutical compositions is described.

**Example 16: Powder for injectable suspension**

**Composition:**

| **INJECTABLE** | **mg/ml** | **%** |
|---|---|---|
| Active ingredient (Compound of Formula I) | 10 | 1 |
| Methylparaben | 1 | 0.1 |
| Prophylparaben | 0.1 | 0.01 |
| Propyleneglycol | 100 | 10 |
| Sodium metabisulfite | 0.25 | 0.025 |
| Sodium chloride | 8.5 | 0.85 |
| Water for injection | csp | |

**Manufacturing Process:**

Powder for suspension

- Fill the active ingredient into vials

Diluent

- Dissolve methylparaben, prophylparaben, sodium metabilsulfite, sodium chloride in propyleneglycol. Mix for a suitable time
- Add water for injection and mix for a suitable time.
- Sterilise by filtration and fill into vials

For final reconstitution before administration, put the diluent solution into the active ingredient vial and shake up to homogeneization.

A list of suitable excipients for injectable suspensions has been detailed above.

## Example 17: Film coated tablet

**Composition:**

| **FILM COATED TABLETS** | **mg / tablet** | **% (over FCT)** |
|---|---|---|
| **Tablet Core:** | | |
| Active ingredient (Compound of Formula I) | 400 | 55.5 |
| Microcrystalline cellulose | 136 | 18.9 |
| Povidone K-25 | 5 | 0.7 |
| Maize starch | 10 | 1.4 |
| Maize Starch, pregelatinised | 25 | 3.5 |
| Lactose | 100 | 13.9 |
| Sodium croscarmellose | 7 | 1.0 |
| Talc | 12 | 1.7 |
| Magnesium stearate | 5 | 0.7 |
| Total (core) | 700 | |
| **Film coating:** | | |
| Hypromellose | 8 | 1.1 |
| Titanium dioxide | 5 | 0.7 |
| Macrogol/PEG 4000 | 3 | 0.4 |
| Lactose | 4 | 0.6 |
| Total (tablet) | 720 | |

**Manufacturing Process:**

- Prepare granulation solution dissolving Povidone K-25 in water
- Mix the active ingredient, maize starch, maize starch pregelatinised and microcrystalline cellulose.
- Granulate with granulation solution
- Drying
- Sieve the dried granules through a suitable mesh size
- Add lactose, sodium croscarmellose and talc
- Mix for a suitable time
- Add magnesium stearate
- Mix for a suitable time
- Once final blend is finished, then is ready for tabletting.
- Tablet compression
- Film coating

A list of suitable excipients for coated tablets has been detailed above.

**Example 18: Powder for oral solution (POS) in sachet**

**Composition:**

| POS-SACHETS | mg / sachet | % |
|---|---|---|
| Active ingredient (Compound of Formula I) | 500 | 9.9% |
| Citric acid | 150 | 3.0% |
| Sodium citrate | 100 | 2.0% |
| Manitol | 2500 | 49.5% |
| Sorbitol | 1500 | 29.7% |
| Sucralose | 150 | 3.0% |
| Aerosil 200 | 5 | 0.1% |
| Lemon flavour | 75 | 1.5% |
| Cola flavour | 75 | 1.5% |
| Total | 5,055 | |

**Manufacturing Process:**

- Pass all the components through a suitable mesh size
- Mix the components into a suitable mixer
- Discharge final blend into containers
- Dosage the blend into sachets

A list of suitable excipients for powders for oral solution has been detailed above.

**Example 19: Syrup**

**Composition:**

| SYRUP | mg/ml | % |
|---|---|---|
| Active ingredient (Compound of Formula I) | 5 | 0.5 |
| Propyleneglycol | 50 | 5 |
| Ethanol | 10 | 1 |
| Sorbitol | 250 | 25 |
| Sodium benzoate | 1.5 | 0.15 |
| Citric acid | 20 | 2 |
| Sodium citrate | 15 | 1.5 |
| Vainille flavour | 1.2 | 0.12 |
| Tartrazine | 30 | 3 |

(continued)

| SYRUP | mg/ml | % |
|---|---|---|
| Purified water | csp | |
| Total | 380 | |

**Manufacturing Process:**

- Put propyleneglycol and ethanol in a suitable container
- Add sodium benzoate up to total dissolution
- Add citric acid and sodium citrate and mix up to total dissolution
- Add active ingredient and mix up to homogeneization
- Add sorbitol and mix up to homogeneization
- Add purified water and mix up to homogeneization
- Add vainille flavour and tartrazine and mix up to homogeneization
- Once the syrup bulk is finished, it is ready for dosing into glass or plastic bottles.

A list of suitable excipients for syrups has been detailed above.

**Example 20: Capsules**

**Composition:**

| CAPSULES | mg / capsule | % |
|---|---|---|
| Active ingredient (Compound of Formula I) | 400 | 66.7 |
| Microcrystalline cellulose | 85 | 14.2 |
| Calcium phosphate | 100 | 16.7 |
| Talc | 10 | 1.7 |
| Magnesium stearate | 5 | 0.8 |
| Total | 600 | |

**Manufacturing Process:**

- Pass all the components through a suitable mesh size
- Put active ingredient, cellulose microcrystalline, calcium phosphate and talc into a suitable mixer.
- Mix for a suitable time
- Add magnesium stearate

- Mix for a suitable time
- Once the final blend is finished, it is ready to be dosed into gelatin capsules

(suitable size)

A list of suitable excipients for capsules has been detailed above.

**<u>Example xx: Gastrorresistant capsules</u>**

**Composition:**

| GASTRORRESISTANT CAPSULES(pellets) | mg / capsule | % |
|---|---|---|
| Active ingredient (Compound of Formula I) | 40 | 10 |
| Microcrystalline cellulose spheres | 260 | 65 |
| Poloxamer | 15 | 3.8 |
| Copolymer methacrylic acid | 80 | 20.0 |
| Pftalate dibutyl | 2 | 0.5 |
| Erythrosine | 3 | 0.8 |
| Isopropyl alcohol | eliminated during process | |
| Acetone | eliminated during process | |
| Total | 400 | |

**Manufacturing Process:**

- Dissolve erythrosine, phthalate dibutyl and copolymer methacrylic
- acid in acetone + isopropylalcohol.
- Add active ingredient and the poloxamer and dissolve it in the previous solution.
- Put the microcrystalline cellulose spheres into a fluid bed dryer.
- Spray the coating solution over on the cellulose spheres.
- Once the coating solution has been totally sprayed, dry the granules.
- Once dried, discharge into a suitable container
- Fill gelatine capsules with the coated spheres A list of suitable excipients for gastrorresistant capsules has been detailed above.

**Claims**

**1.** A compound of Formula (I):

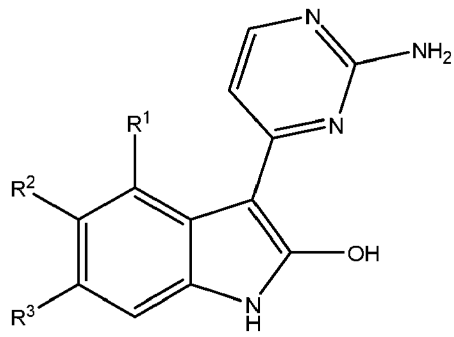

Formula I

(I)

wherein:

$R^1$ is selected from hydrogen, halogen, OH, $NH_2$ and $NO_2$;
$R^2$ is selected from hydrogen, halogen, OH, $NH_2$ and $-NO_2$; and
$R^3$ is selected from hydrogen, halogen, OH, $NH_2$, $NO_2$, $-S(O)_2-R^4$ and $-COOR^4$, wherein $R^4$ is selected from hydrogen and $C_1$-$C_6$ alkyl;
or any pharmaceutically acceptable salt, solvate or prodrug thereof.

**2.** The compound according to claim 1, wherein $R_1$ is hydrogen.

**3.** The compound according to any of claims 1 and 2, wherein $R^1$, $R^2$ and $R^3$ are hydrogen.

**4.** The compound according to any of claims 1 and 2, wherein $R^1$ and $R^3$ are hydrogen and $R^2$ is halogen or $NO_2$.

**5.** The compound according to claim 4, wherein $R^1$ and $R^3$ are hydrogen and $R^2$ is halogen.

**6.** The compound according to any of claims 1 and 2, wherein $R^1$ and $R^2$ are hydrogen and $R^3$ is $-S(O)_2-R^4$ or $-COOR^4$, wherein $R^5$ is selected from hydrogen and $C_1$-$C_6$ alkyl.

**7.** The compound according to claim 6, wherein $R^4$ is methyl.

**8.** The compound according to claim 1, wherein $R^1$ and $R^2$ are halogen and $R^3$ is hydrogen.

**9.** The compound according to any one of claims 1 to 8, selected from the following compounds:

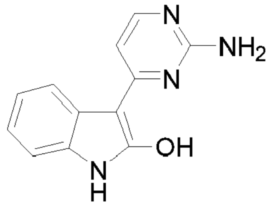

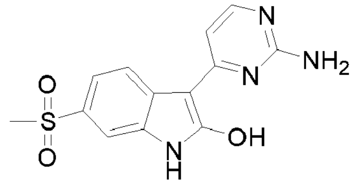

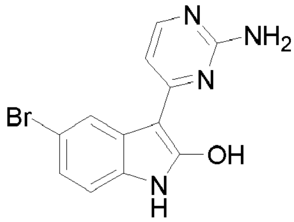

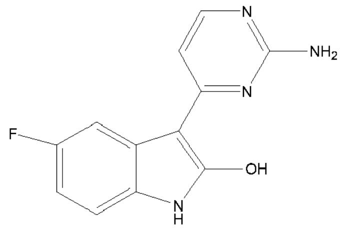

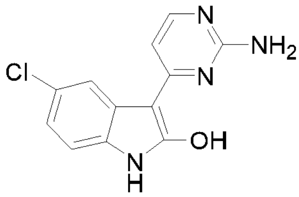

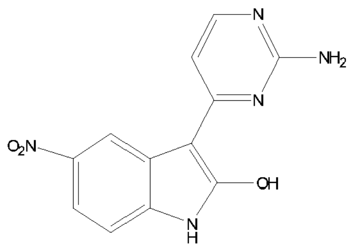

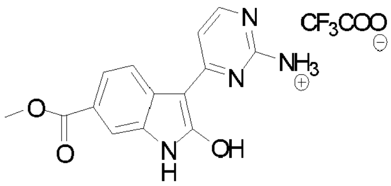

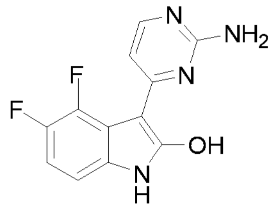

**10.** A process for the preparation of a compound of Formula (I) as defined in any one of claims 1 to 9, which comprises:

a) protecting the amino reactive functional group of a compound of formula (II):

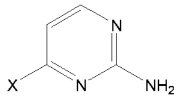

(II)

with a protecting group to form the compound of formula (III):

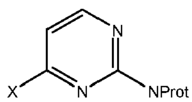

(III)

wherein X is a halogen selected from chloride and bromide and Prot is the protecting group of the amino group;
b) reacting the compound of formula (III) with an oxindole of formula (IV):

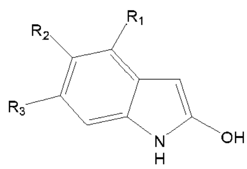

(IV)

wherein:

$R_1$, $R_2$ and $R_3$ are as defined above,
in the presence of a strong base, to form a compound of formula (V):

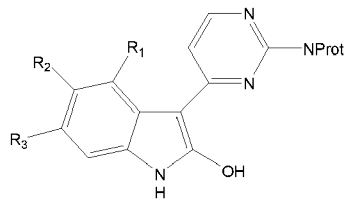

(V)

c) deprotecting the amino group of formula (V) to give the compound of formula (I).
or alternatively,
a) reacting an oxindole of formula (IV):

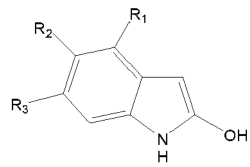

(IV)

with an electrophile of formula (VI):

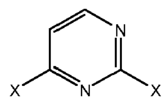

(VI)

to form a compound of formula (VII):

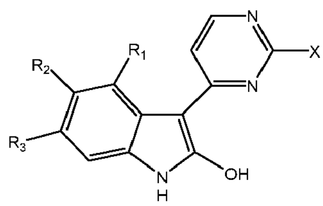

(VII)

and
b) reacting the compound of formula (VII) with ammonium hydroxide to form the compound of formula (I).

**11.** A pharmaceutical composition comprising a compound of Formula (I) as defined in any one of claims 1 to 9, or a salt, solvate or prodrug thereof, and at least one pharmaceutically acceptable carrier, adjuvant, and/or vehicle.

**12.** A compound of Formula (I) as defined in any one of claims 1 to 9, or a salt, solvate or prodrug thereof, for its use as a medicament.

**13.** A compound of Formula (I) as defined in any one of claims 1 to 9, or a salt, solvate or prodrug thereof, for its use in the treatment of a cognitive, neurodegenerative or neurological disease or condition.

**14.** A compound of Formula (I) as defined in any one of claims 1 to 9, or a salt, solvate or prodrug thereof, for its use in the treatment of a disease or condition selected from diabetes, diabetes mellitus, inflammatory and autoimmune diseases, cardiovascular disorders, and pathologies selected from metabolic syndrome X, hair loss, severe acute respiratory syndrome coronavirus, stimulant drugs addiction, cocaine addiction, bone loss and glaucoma.

**15.** A compound of formula (I) as defined in any one of claims 1 to 9, or a salt, solvate or prodrug thereof, as a reactive in an *in vitro* biological assay requiring inhibition of CDK5, GSK-3 or CK1ε.

Europäisches Patentamt
European Patent Office
Office européen des brevets

# EUROPEAN SEARCH REPORT

Application Number
EP 12 38 2133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2005/123672 A2 (TAKEDA SAN DIEGO INC [US]; BRESSI JEROME C [US]; GANGLOFF ANTHONY R [U) 29 December 2005 (2005-12-29) * example 1 * ----- | 1-15 | INV. C07D403/04 A61K31/506 A61P25/00 A61P3/10 |

TECHNICAL FIELDS SEARCHED (IPC)
C07D
A61K
A61P

The present search report has been drawn up for all claims

1

| Place of search | Date of completion of the search | Examiner |
|---|---|---|
| The Hague | 4 July 2012 | de Nooy, Arjan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 12 38 2133

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2012

| Patent document cited in search report | Publication date | Patent family member(s) | Publication date |
|---|---|---|---|
| WO 2005123672 A2 | 29-12-2005 | EP 1773807 A2 | 18-04-2007 |
| | | JP 2008502687 A | 31-01-2008 |
| | | US 2008153869 A1 | 26-06-2008 |
| | | WO 2005123672 A2 | 29-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005123672 A


### Non-patent literature cited in the description


- *Nat. Rev., Mol. Cell Biol.,* 2001, vol. 2, 749-759
- **DHAVAN R. ; TSAI L.H.** *Journal of Neurochemistry,* 2004, vol. 88, 1313-1326
- **SHELTON S.B. ; JOHNSON G. V. W.** *Cell Mol Neurobiol,* 2008, vol. 28, 351-369
- **DHAVAN R. ; TSAI L.H.** A decade of CDK5. *Nat. Rev., Mol. Cell Biol.,* 2001, vol. 2, 749-759
- **TSAI L.H. ; LEE M.S. ; CRUZ J.** Cdk5, a therapeutic target for Alzheimer's disease?. *Biochimica et Biophysica Acta,* 2004, vol. 1697, 137-142
- *Cell Mol Neurobiol,* 2008, vol. 28, 351-369
- **DHARIWALA F.A. ; RAJADHYAKSHA M.S.** An Unusual Member of the Cdk Family: Cdk5. *J. Comp. Neurol.,* 1999, vol. 415, 218-229
- **KWON Y.T. ; TSAI L.H. ; CRANDALL J.E.** Callosal axon guidance defects in p35 (-/-) mice. *Journal of Neurochemistry,* 2004, vol. 88, 1313-1326
- **SHELTON S.B. ; JOHNSON G.V.W.** Cyclin-dependent kinase-5 in neurodegeneration. *Journal of Neurochemistry,* vol. 88, 1313-1326
- **TSAI L.H. ; LEE M.S. ; CRUZ J.** Cdk5, a therapeutic target for Alzheimer's disease?. *Biochimica et Biophysica Acta,* 2004, vol. 1697, 137-142
- **TSAI L. H.** Activity and expression pattern of cyclin-dependent kinase 5 in the embryonic mouse nervous system. *Development,* 2003, vol. 119, 1029-1040
- **LEW.** A brain-specific activator of cyclin-dependent kinase 5. *Nature,* 1994, vol. 371, 423-426
- **TSAI.** p35 is a neural-specific regulatory subunit of cyclin-dependent kinase 5. *Nature,* 1994, 3-71419-423
- **UCHIDA.** Precursor of cdk5 activator, the 23 kDa subunit of tau protein kinase II: its sequence and developmental change in brain. *FEBS Lett.,* 1994, vol. 355, 35-40
- **TANG.** An isoform of the neuronal cyclin-dependent kinase 5 (Cdk5) activator. *J. Biol. Chem.,* 1995, vol. 270, 26897-26903
- **JEONG.** The cyclin-dependent kinase 5 activator, p39, is expressed in stripes in the mouse cerebellum. *Neuroscience,* 2003, vol. 118, 323-334
- **TSAI L.H.** The role of cyclin-dependent kinase 5 in brain development and degeneration. *Cell. Mol. Biol. Lett.,* 2003, vol. 8, 546, 547
- **DHARIWALA F.A. ; RAJADHYAKSHA M.S.** An Unusual Member of the Cdk Family: Cdk5. *Cell Mol Neurobiol,* 2008, vol. 28, 351-369
- **KWON Y.T. ; TSAI L.H. ; CRANDALL J. E.** Callosal axon guidance defects in p35 (-/-) mice. *J.Comp. Neurol.,* 1999, vol. 415, 218-229
- **ZHANG M.** Cyclin-Dependent Kinase Inhibitors Attenuate Protein Hyperphosphorylation, Cytoskeletal Lesion Formation, and Motor Defects in Niemann-Pick Type C Mice. *American Journal of Pathology,* 2004, vol. 165 (3), 843-853
- **TOWN T.** p35/ Cdk5 pathway mediates soluble amyloid-beta peptide-induced tau phosphorylation in vitro. *J. Neurosci. Res.,* 2002, vol. 69, 362-372
- **IMAHORI K. ; UCHIDA T.** Physiology and pathology of tau protein kinases in relation to Alzheimer's disease. *J. Biochem. (Tokyo),* 1997, vol. 121, 179-188
- **NOBLE W.** Cdk5 Is a Key Factor in Tau Aggregation and Tangle Formation In Vivo. *Neuron,* 2003, vol. 38, 555-565
- **SHELTON S.B. ; JOHNSON G.V.W.** Cyclin-dependent kinase-5 in neurodegeneration. *Journal of Neurochemistry,* 2004, vol. 88, 1313-1326
- **CRUZ J.C.** Aberrant Cdk5 Activation by p25 Triggers Pathological Events Leading to Neurodegeneration and Neurofibrillary Tangles. *Neuron,* 2003, vol. 40, 471-483
- **WEN, Y.** Interplay between Cyclin-Dependent Kinase 5 and Glycogen Synthase Kinase 3β Mediated by Neuregulin Signaling Leads to Differential Effects on Tau Phosphorylation and Amyloid Precursor Protein Processing. *J. Neurosci.,* 2008, vol. 28, 2624-2632
- **TSAI L.H., ; LEE M.S. ; CRUZ J.** Cdk5, a therapeutic target for Alzheimer's disease?. *Biochimica et Biophysica Acta,* 2004, vol. 1697, 137-142
- **BAJAJ.** Cyclin-dependent kinase-5 is associated with lipofuscin in motor neurones in amyotrophic lateral sclerosis. *Neurosci. Lett.,* 1998, vol. 245, 45-48

- **BU B.** Niemann-Pick disease type C yields possible clue for why cerebellar neurons do not form neurofibrillary tangles. *Neurobiol Dis,* 2002, vol. 11, 285-97
- **RAJGOPAL Y. ; VEMURI M.C.** Ethanol induced changes in cyclin-dependent kinase-5 activity and its activators, P35, P67 (Munc-18) in rat brain. *Neurosci. Lett.,* 2001, vol. 308, 173-176
- **BRION J.P. ; COUCK A.M.** Cortical and brainstem-type Lewy bodies are immunoreactive for the cyclin-dependent kinase 5. *Am. J. Pathol.,* 1995, vol. 147, 1465-1476
- **SMITH P.D.** Cyclin-dependent kinase 5 is a mediator of dopaminergic neuron loss in a mouse model of Parkinson's disease. *Proc Natl Acad Sci U S A,* 2003, vol. 100, 13650-13655
- **. BORGHI R.** Increase of cdk5 is related to neurofibrillary pathology in progressive supranuclear palsy. *Neurology,* 2002, vol. 58, 589-592
- **HAN D.** Familial FTDP-17 missense mutations inhibit microtubule assembly-promoting activity of tau by increasing phosphorylation at Ser202 in vitro. *J Biol Chem.,* 2009, vol. 284 (20), 13422-33
- **SULTANA R. ; BUTTERFIELD D.A.** Regional expression of key cell cycle proteins in brain from subjects with amnestic mild cognitive impairment. *Neurochem Res.,* 2007, vol. 32 (4-5), 655-62
- **POLLONINI G.** Abnormal expression of synaptic proteins and neurotrophin-3 in the Down syndrome mouse model Ts65Dn. *Neuroscience,* 2008, vol. 22, 156 (1), 99-106
- Dopaminergic and glutamatergic signaling crosstalk in Huntington's disease neurodegeneration: the role of p25/cyclin-dependent kinase 5. *J Neurosci.,* 2008, vol. 28 (40), 10090-101
- **SCHNEIDER A.** Hyperphosphorylation and aggregation of tau in experimental autoimmune encephalomyelitis. *J Biol Chem.,* 2004, vol. 279 (53), 55833-9
- **OSUGA H.** Cyclin-dependent kinases as a therapeutic target for stroke. *Proc Natl Acad Sci USA,* vol. 97, 10254-10259
- **WANG J.** Cdk5 activation induces hippocampal CA1 cell death by directly phosphorylating NMDA receptors. *Nature Neuroscience,* 2003, vol. 6, 1039-1047
- **SHELTON S.B. ; JOHNSON G.V.W.** Cyclin-dependent kinase-5 in neurodegeneration. *J Neurochem,* 2004, vol. 88 (6), 1313-1326
- **BIBB J.A.** Effects of chronic exposure to cocaine are regulated by the neuronal protein Cdk5. *Nature,* 2001, vol. 410, 376-380
- **. CHEN J.** Induction of Cyclin-Dependent Kinase 5 in the Hippocampus by Chronic Electroconvulsive Seizures: Role of ΔFosB. *J. Neurosci.,* 2000, vol. 20, 8965-8971
- **SEN A.** The potential role of cyclin-dependent kinase 5 in focal cortical dysplasia. *Dev Neurosci.,* 2008, vol. 30 (1-3), 96-104
- **GREEN S.** Alterations in Cyclin-Dependent Protein Kinase 5 (CDK5) Protein Levels, Activity and Immunocytochemistry in Canine Motor Neuron Disease. *J. Neuropathol. Exp. Neurol.,* 1998, vol. 57 (11), 991-1099
- **NAKANO S.** Aberrant expression of cyclin-dependent kinase 5 in inclusion body myositis. *Neurology,* 1999, vol. 53, 1671-1676
- **NAKAMURA S.** Cyclin-dependent kinase 5 and mitogen-activated protein kinase in glial cytoplasmic inclusions in multiple system atrophy. *J. Neuropathol. Exp. Neurol.,* 1998, vol. 57, 690-698
- **FU W.Y.** Induction of Cdk5 activity in rat skeletal muscle after nerve injury. *Neuroreport.,* 2002, vol. 13, 243-247
- **BIGNANTE E.A.** Previous stress exposure enhances both anxiety-like behaviour and p35 levels in the basolateral amygdala complex: Modulation by midazolam. *Eur Neuropsychopharmacol.,* 2010, vol. 20 (6), 388-397
- **XI Z. Q.** Gene expression analysis on anterior temporal neocortex of patients with intractable epilepsy. *Synapse,* 2009, vol. 63 (11), 1017-1028
- **PARESA N.** Phosphorylation of Prion Protein at Serine 43 Induces Prion Protein Conformational Change. *J Neurosci.,* 2009, vol. 29 (27), 8743-8751
- **CHEUNG K.J.** Tyrosine hydroxylase expression and Cdk5 kinase activity in ataxic cerebellum. *Mol Cell Biochem.,* 2008, vol. 318 (1-2), 7-12
- **MIZUNO K.** Expression of p25 impairs contextual learning but not latent inhibition in mice. *Neuroreport.,* 2006, vol. 17 (18), 1903-5
- **TEJ K.** Cdk5: A New Player in Pain Signaling. *Cell Cycle,* 2006, vol. 5 (6), 585-588
- **YASUDA H.** New trend in pathogenesis of diabetic neuropathy. *Rinsho Shinkeigaku.,* 1999, vol. 39 (1), 87-9
- **WEI F.Y. ; TOMIZAWA K.** Cyclin-dependent kinase 5 (Cdk5): a potential therapeutic target for the treatment of neurodegenerative diseases and diabetes mellitus. *Mini Rev Med Chem.,* 2007, vol. 7 (10), 1070-4
- **COGHLAN.** Selective small molecule inhibitors ofglycogen synthase kinase-3 modulate glycogen metabolism and gene transcription. *Chemistry & Biology,* 2000, vol. 7 (10), 793-803
- **KIM, L. ; KIMMEL, A.R.** GSK3, a master switch regulating cell-fate specification and tumorigenesis. *Curr. Opinion Genetics Dev.,* 2000, vol. 10 (5), 508-514
- **ÁVILA, J.** Role of tau protein in both physiological and pathological conditions. *Physiol. Rev.,* 2004, vol. 84, 361-84

- **JUHASZOVA M.** Role of glycogen synthase kinase-3beta in cardioprotection. *Circ Res.,* 2009, vol. 104 (11), 1240-52
- **MIURA T. ; MIKI T.** GSK-3beta, a therapeutic target for cardiomyocyte protection. *Circ J.,* 2009, vol. 73 (7), 1184-92
- **ELDAR-FINKELMAN, H.** Glycogen synthase kinase 3: an emerging therapeutic target. *Trends. Mol. Med.,* 2002, vol. 8, 126-32
- **ASENJO, A.** Residues in human respiratory syncytial virus P protein that are essential for its activity on RNA viral synthesis. *Virus Res.,* 2008, vol. 132, 160-73
- **MEDINA M. ; ÁVILA J.** Glycogen Synthase Kinase-3 (GSK-3) Inhibitors For The Treatment of Alzheimer's Disease. *Current Pharmaceutical Design,* 2010, vol. 16 (25), 2790-2798
- **HERNÁNDEZ F.** The role of GSK3 in Alzheimer disease. *Brain Res Bull.,* 2009, vol. 80 (4-5), 248-50
- **FORLENZA O.V.** Increased platelet GSK3B activity in patients with mild cognitive impairment and Alzheimer's disease. *J Psychiatr Res.,* 2011, vol. 45 (2), 220-4
- **MASAHIRO N. ; HIDEAKI H.** Glycogen synthase kinase-3beta is associated with Parkinson's disease. *Neuroscience Letters,* 2009, vol. 449 (2), 103-107
- **SCHAFFER, B.** Association of GSK3B with Alzheimer disease and frontotemporal dementia. *Arch. Neurol.,* 2008, vol. 65, 1368-74
- **HANGER D.P. ; NOBLE W.** Functional implications of glycogen synthase kinase-3-mediated tau phosphorylation. *Int J Alzheimers Dis.,* 2011, 352805
- **MEDINA, M., ; GARRIDO, J.J. ; WANDOSELL, F.** Modulation of GSK-3 as a therapeutic strategy on taupathologies. *Frontiers in Molecular Neurosciences,* 2011, vol. 4 (24), 1-10
- **BUÉE, L.** Tau protein isoforms, phosphorylation and role in neurodegenerative disorders. *Brain Research Reviews,* 2000, vol. 33, 95-130
- **DEWHURST S.** *J Neuroimmune Pharmacol.,* 2007, vol. 2 (1), 93-96
- **CARMICHAEL J.** Glycogen synthase kinase-3beta inhibitors prevent cellular polyglutamine toxicity caused by the Huntington's disease mutation. *J Biol Chem.,* 2002, vol. 277 (37), 33 791-8
- **TANJI K.** Glycogen synthase kinase-3beta phosphorylates synphilin-1 in vitro. *Neuropathology,* 2003, vol. 23 (3), 199-202
- **ROEW M.K.** GSK-3 is a viable potential target for therapeutic intervention in bipolar disorder. *Neurosci Biobehav Rev.,* 2007, vol. 31 (6), 920-931
- **LI X.** Glycogen Synthase Kinase-3$\beta$, mood stabilizers, and neuroprotection. *Bipolar Disord.,* 2002, vol. 4 (2), 137-144
- **WADA A.** Lithium and neuropsychiatric therapeutics: neuroplasticity via glycogen synthase kinase-3beta, beta-catenin, and neurotrophin cascades. *J Pharmacol Sci.,* 2009, vol. 110 (1), 14-28
- **KOROS E. ; DORNER-CIOSSEK C.** The role of glycogen synthase kinase-3beta in schizophrenia. *Drug News Perspect.,* 2007, vol. 20 (7), 437-45
- **LOVESTONE S.** Schizophrenia as a GSK-3 dysregulation disorder. *Trends Neurosci.,* 2007, vol. 30 (4), 142-9
- **CHEN G.** The mood-stabilizing agent valproate inhibits the activity of glycogen synthase kinase-3. *J Neurochem.,* 1999, vol. 72 (3), 1327-30
- **JOPE R. S. ; ROH M.S.** Glycogen synthase kinase-3 (GSK3) in psychiatric diseases and therapeutic interventions. *Curr Drug Targets.,* 2006, vol. 7 (11), 1421-34
- **BEAULIEU J.M.** Role of GSK3 beta in behavioral abnormalities induced by serotonin deficiency. *Proc Natl Acad Sci U S A.,* 2008, vol. 105 (4), 1333-8
- **BEAULIEU J.M.** Lithium antagonizes dopamine-dependent behaviors mediated by an AKT/glycogen synthase kinase 3 signaling cascade. *Proc Natl Acad Sci U S A.,* 2004, vol. 101 (14), 5099-104
- **LIU F.** Overexpression of Dyrk1A contributes to neurofibrillary degeneration in Down syndrome. *FASEB J.,* 2008, vol. 22 (9), 3224-33
- **YUSKAITIS C.J.** Lithium ameliorates altered glycogen synthase kinase-3 and behavior in a mouse model of fragile X syndrome. *Biochem Pharmacol,* 2010, vol. 79 (4), 632-46
- **. DUGO L.** Glycogen synthase kinase 3beta as a target for the therapy of shock and inflammation. *Shock.,* 2007, vol. 27 (2), 113-23
- **SASAKI C.** Different expression of glycogen synthase kinase-3beta between young and old rat brains after transient middle cerebral artery occlusion. *Neurol Res.,* 2001, vol. 23 (6), 588-92
- **BEUREL E.** Innate and adaptive immune responses regulated by glycogen synthase kinase-3 (GSK3). *Trends Immunol.,* 2010, vol. 31 (1), 24-31
- **DE SARNO P.** Lithium prevents and ameliorates experimental autoimmune encephalomyelitis. *J. Immunol.,* 2008, vol. 181 (1), 338-45
- **WATASE K.** Lithium therapy improves neurological function and hippocampal dendritic arborization in a spinocerebellar ataxia type 1 mouse model. *PLoS Med,* 2007, vol. 4 (5), 836-847
- **HAN F.** Accumulation of beta-amyloid in the brain microvessels accompanies increased hyperphosphorylated tau proteins following microsphere embolism in aged rats. *Neuroscience,* 2008, vol. 153 (2), 414-27
- **YANG W.** Upregulation of GSK3$\beta$ expression in frontal and temporal cortex in ALS with cognitive impairment (ALSci). *Brain Res.,* 2008, vol. 1196, 131-139

- **PÉREZ M.** Prion peptide induces neuronal cell death through a pathway involving glycogen synthase kinase 3. *Biochem J.,* 2003, vol. 372, 129-36
- **WANG G.R.** Changes of tau profiles in brains of the hamsters infected with scrapie strains 2 63 K or 139 A possibly associated with the alteration of phosphate kinases. *BMC Infect Dis.,* 2010, vol. 1 (10), 86
- **YUAN Y. E.** Overexpressed alpha-synuclein regulated the nuclear factor-kappaB signal pathway. *Cell Mol Neurobiol.,* 2008, vol. 28 (1), 21-33
- **JIN J.** GSK3beta-cyclin D3-CUGBP1-eIF2 pathway in aging and in myotonic dystrophy. *Cell Cycle,* 2009, vol. 8 (15), 2356-9
- **DILL, J.** Inactivation of glycogen synthase kinase 3 promotes axonal growth and recovery in the CNS. *J. Neurosci.,* 2008, vol. 28, 8914-28
- **COHEN P.** The role ofprotein phosphorylation in human health and disease. *Eur J Biochem.,* 2001, vol. 268 (19), 5001-10
- **CUZZOCREA, S.** Glycogen synthase kinase-3b inhibition attenuates the degree of arthritis caused by type II collagen in the mouse. *Clin. Immunol.,* 2006, vol. 120, 57-67
- **HU, X.** IFN-g suppresses IL-10 production and synergizes with TLR2 by regulating GSK3 and CREB/AP-1 proteins. *Immunity,* 2006, vol. 24, 563-574
- **DUGO, L.** GSK-3b inhibitors attenuate the organ injury/dysfunction caused by endotoxemia in the rat. *Crit. Care Med.,* 2005, vol. 33, 1903-1912
- **BENTLEY J.K.** Airway smooth muscle hyperplasia and hypertrophy correlate with glycogen synthase kinase-3(beta) phosphorylation in a mouse model of asthma. *Am JPhysiol Lung Cell Mol Physiol.,* 2009, vol. 296 (2), L176-84
- GSK-3b inhibitors reduce protein degradation in muscles from septic rats and in dexamethasone treated myotubes. *J. Biochem. Cell. Biol.,* 2005, vol. 37, 2226-2238
- **A.R.** *Int. Evenson*
- **WHITTLE, B.J.** Reduction of experimental colitis in the rat by inhibitors of glycogen synthase kinase-3b. *Br. J. Pharmacol.,* 2006, vol. 147, 575-582
- **DUGO, L.** Glycogen synthase kinase-3b inhibitors protect against the organ injury and dysfunction caused by hemorrhage and resuscitation. *Shock,* 2006, vol. 25, 485-491
- **GONG R.** Glycogen synthase kinase 3beta: a novel marker and modulator of inflammatory injury in chronic renal allograft disease. *Am J Transplant.,* 2008, vol. 8 (9), 1852-63
- **LENZ, S.P.** Lithium chloride enhances survival of NZB/W lupus mice: influence of melatonin and timing of treatment. *Int. J. Immunopharmacol.,* 1995, vol. 17, 581-592
- **HARDT, S.E. ; SADOSHIMA, J.** Glycogen synthase kinase-3beta: a novel regulator of cardiac hypertrophy and development. *Circ. Res.,* 2002, vol. 90, 1055-63
- **BOWES A.J.** Valproate attenuates accelerated atherosclerosis in hyperglycemic apoE-deficient mice: evidence in support of a role for endoplasmic reticulum stress and glycogen synthase kinase-3 in lesion development and hepatic steatosis. *Am J Pathol.,* 2009, vol. 174 (1), 330-42
- **BADORFF C.** Fas receptor signaling inhibits glycogen synthase kinase 3β and induces cardiac hypertrophy following pressure overload. *J. Clin. Invest.,* 2002, vol. 109 (3), 373-381
- **MA X. E.** Epub. Delayed Re-endothelialization with Rapamycin-coated Stents is Rescued by the Addition of a Glycogen Synthase Kinase 3 Beta Inhibitor. *Cardiovasc Res.,* 2010
- **GALLICCHIO, V. S.** Lithium and the Cell. 1991, 185-198
- **WAGMAN A.S.** Discovery and development of GSK3 inhibitors for the treatment of type 2 diabetes. *Curr Pharm Des.,* 2004, vol. 10 (10), 1105-37
- **LYUBIMOVA A.** Neural Wiskott-Aldrich syndrome protein modulates Wnt signaling and is required for hair follicle cycling in mice. *J Clin Invest.,* 2010, vol. 120 (2), 446-56
- **WU C.H.** Glycogen synthase kinase-3 regulates the phosphorylation of severe acute respiratory syndrome coronavirus nucleocapsid protein and viral replication. *J Biol Chem.,* 2009, vol. 284 (8), 5229-39
- **XU C.M.** Glycogen synthase kinase 3beta in the nucleus accumbens core mediates cocaine-induced behavioral sensitization. *J Neurochem.,* 2009, vol. 111 (6), 1357-68
- **WANG F.S.** Inhibition of glycogen synthase kinase-3beta attenuates glucocorticoid-induced bone loss. *Life Sci.,* 2009, vol. 85 (19-20), 685-92
- **WANG W.H.** Increased expression of the WNT antagonist sFRP-1 in glaucoma elevates intraocular pressure. *J Clin Invest.,* 2008, vol. 118 (3), 1056-64
- **COHEN, P. ; GOEDERT, M.** GSK3 inhibitors: development end therapeutic potential. *Nature Reviews,* 2004, vol. 3, 479-487
- **HERNÁNDEZ, F.** GSK3 Inhibitors and Disease. *Mini-Reviews in Medicinal Chemistry,* 2009, vol. 9 (9), 1024-1029
- **MEDINA, M. ; CASTRO, A.** Glycogen synthase kinase-3 (GSK-3) inhibitors reach the clinic. *Curr. Opin. Drug Discov. Develop.,* 2008, vol. 11 (4), 533-543
- Glycogen Synthase Kinase 3 (GSK-3) and its inhibitors. John Wiley & Sons, Inc.,, 2006

- **LECLERC, S.** Indirubins inhibit glycogen synthase kinase-3 beta and CDK5lp25, two protein kinases involved in abnormal tau phosphorylation in Alzheimer's disease. A property common to most cyclin-dependent kinase inhibitors?. *J. Biol. Chem.,* 2001, vol. 276, 251-60
- **SMITH, D.** 3-Anilino- 4-arylmaleimides: potent and selective inhibitors of glycogen synthase kinase-3 (GSK-3). *Bioorg. Med. Chem. Lett.,* 2001, vol. 11, 635-9
- **WITHERINGTON, J. E.** 5-arylpyrazolo[3,4-b]pyridazines: potent inhibitors of glycogen synthase kinase-3 (GSK-3). *Bioorg. Med. Chem. Lett.,* 2003, vol. 13, 1581-4
- **LEOST, M.** Paullones are potent inhibitors of glycogen synthase kinase-3beta and cyclin-dependent kinase 5/p25. *Eur. J. Biochem.,* 2000, vol. 267, 5983-94
- **BHAT, R.** Structural insights and biological effects of glycogen synthase kinase 3-specific inhibitor AR-A014418. *J. Biol. Chem.,* 2003, vol. 278, 45937-45
- **MARTINEZ, A.** First non-ATP competitive glycogen synthase kinase 3 beta (GSK-3beta) inhibitors: thiadiazolidinones (TDZD) as potential drugs for the treatment of Alzheimer's disease. *J. Med. Chem.,* 2002, vol. 45, 1292-9
- **OSPINA ; FERNANDEZ-RENART.** *Biochim. Biophys. Acta,* 1990, vol. 1052, 483
- **TUAZON ; TRAUGH.** *Adv. Second Messenger Phosphoprot.Res.,* 1991, vol. 23, 123
- **KLIMCZAK ; CASHMORE.** *Biochem. J,* 1993, vol. 293, 283
- **WALCZAK et al.** *Biochem. J,* 1993, vol. 296, 729
- **PULGAR et al.** *Eur. J. Biochem.,* 1996, vol. 242, 519
- **PETERS et al.** *Nature,* 1999, vol. 401, 345
- **LIU et al.** *Plant J.,* 2003, vol. 36, 189
- **KNIPPSCHILD et al.** *Cellular Signalling,* 2005, vol. 17, 675-689
- **FLAJOLET et al.** *PNAS,* 2007, vol. 104 (10), 4159-4164
- **PÉREZ D.I. et al.** *Med Res Rev.,* 2010
- **LAMPE P.D. ; LAU A.F.** *Int J Biochem Cell Biol,* 2004, vol. 36, 1171-1186
- **KNIPPSCHILD U. et al.** *Onkologie,* 2005, vol. 28, 508-514
- **ROBINSON L.C. et al.** *Mol Cell Biol,* 1993, vol. 13, 2870-2881
- **PRICE M.A.** *Genes Dev.,* 2006, vol. 20, 399-410
- **EIDE E.J. ; VIRSHUP D.M.** *Chronobiol Int.,* 2001, vol. 18, 389-398
- **EBISAWA T.** *J Pharmacol Sci,* 2007, vol. 103, 150-154
- **MENG Q.J. et al.** *Neuron,* 2008, vol. 58, 78-88
- **GROSS ; ANDERSON.** *Cell Signalling,* 1998, vol. 10, 699-711
- **WANG et al.** *Mol. Biol. Cell,* 1992, vol. 3, 275
- **VANCURA et al.** *J Biol. Chem.,* 1994, vol. 269, 19271
- **HO et al.** *Proc. Natl. Acad. Sci. U. S. A.,* 1997, vol. 94, 581
- **DEMAGGIO et al.** *Proc. Natl. Acad. Sci. U. S. A.,* 1992, vol. 89, 7008
- **GRAVES et al.** *J. Biol. Chem.,* 1993, vol. 268, 6394
- **CARMEL et al.** *J. Biol. Chem.,* 1994, vol. 269, 7304
- **FISH et al.** *J Biol. Chem.,* 1995, vol. 270, 14875
- **ZHAI et al.** *J Biol.Chem.,* 1995, vol. 270, 12717
- **GRAVES ; ROACH.** *J. Biol. Chem.,* 1995, vol. 270, 21689
- **GRAVES et al.** *J Biol. Chem.,* 1993, vol. 268, 6394
- **LONGENECKER et al.** *Acta Crystallogr., D Biol. Crystallogr.,* 1998, vol. 54, 473
- **RIVERS A. et al.** *J Biol. Chem.,* 1998, vol. 273, 15980
- **GIETZEN KF ; VIRSHUP DM.** *JBiol Chem.,* 1999, vol. 274, 32063-32070
- **GROSS ; ANDERSON.** *Cell Signal,* 1998, vol. 10, 699-711
- **VANCURA et al.** *J. Biol. Chem.,* 1994, vol. 269, 19271
- **ISSINGER.** *Pharmacol Ther,* 1993, vol. 59, 1-30
- **GROSS et al.** *J Cell Biol,* 1995, vol. 130, 711-724
- **DESDOUITS et al.** *Proc Natl Acad Sci USA,* 1995, vol. 92, 2682-2685
- **SINGH et al.** *J Neurochem,* 1995, vol. 64, 1420-1423
- **KLOSS et al.** *Neuron,* 2001, vol. 30, 699-706
- **SAKANAKA et al.** *PNAS,* 1999, vol. 96 (22), 12548-12552
- **KURET et al.** *J. Neurochem.,* 1997, vol. 69, 2506-2515
- **GHOSHAL et al.** *Am. J. Pathol.,* 1999, vol. 155, 1163-1172
- **JELLINGER K.A. ; BANCHER C.** *J. Neural Transm,* 1998, vol. 54, 77-95
- **VIERLHABER ; VIRSHUP.** *IUBMB Life,* 2001, vol. 51, 73-78
- **CHAUHAN A. et al.** *Brain Res.,* 1993, vol. 629, 47-52
- **GRUNDKE-IGBAL I. et al.** *J. Biol. Chem.,* 1986, vol. 261, 6084-6089
- **SINGH et al.** *Mol. Cell. Bioch.,* 1994, vol. 131, 181-9
- **SINGH et al.** *JNeurochem,* 1995, vol. 64, 1420-3

- **MEIJER et al.** *Trends Pharmacol Sci.,* 2004, vol. 25, 471-480
- **LIU et al.** *Proc Natl Acad Sci USA,* 2001, vol. 98, 11062-11068
- **WALTER et al.** *J Biol Chem,* 2001, vol. 276, 14634-14641
- **WALTER et al.** *Biochemistry,* 1998, vol. 37, 5961-5967
- **WALTER et al.** *J. Biol. Chem.,* 2001, vol. 276 (18), 14634-14641
- **CHERGI K. et al.** *J. Neurosci.,* 2005, vol. 25 (28), 6601-6609
- **WAXMAN E.A ; GIASSON B.I.** *J Neuropathol Exp Neurol.,* 2008, vol. 67 (5), 402-416
- **SINGH,T.J. et al.** *J. Neurochem.,* 1995, vol. 64 (3
- **SCHWAB C. et al.** *Neurobiology of Aging,* 2000, vol. 21, 503-510
- **FERRER I. et al.** *Current Alzheimer Research,* 2005, vol. 2, 3-18
- **CHURCHER, I.** *Current Topics in Medicinal Chemistry,* 2006, vol. 6 (6), 579-595
- **ENGELBERG H.** *Dement. Geriatr. Cogn. Disord.,* 2004, vol. 18, 278-298
- **LARSON E.B. et al.** *Annu. Rev. Publ. Health,* vol. 13, 431-49
- **HASEGAWA M. et al.** *Ann Neurol.,* 2008, vol. 64 (1), 60-70
- **MACLAINE N.J. ; HUPP T.R.** *Aging (Albany NY),* 2009, vol. 1 (5), 490-502
- **VEENSTRA-VANDERWEELE J. et al.** *Neuropsychopharmacology,* 2006, vol. 31, 1056-1063
- **YINAN M. et al.** *Neuroscience Letters,* 2004, vol. 368 (1), 2-6
- **SHINODA S. et al.** *Journal of Neurochemistry,* 2003, vol. 86, 460-469
- **WALTON K.M. et al.** *The Journal of Pharmacology and Experimental Therapeutics,* 2009, vol. 330, 430-439
- **SAKURAI E. et al.** *Molecular Pain,* 2009, vol. 5, 74
- **IQBAL, K.** Mechanisms of tau-induced neurodegeneration. *Acta Neuropathol.,* 2009, vol. 118 (1), 53-69
- **COMPTA, Y.** Cerebrospinal tau, phospho-tau, and beta-amyloid and neuropsychological functions in Parkinson's disease. *Mov Disord.,* 2009, vol. 24 (15), 2203-10
- **BREW, B.J.** CSF amyloid beta42 and tau levels correlate with AIDS dementia complex. *Neurology,* 2006, vol. 65 (9), 1490-2
- **MANN, D.M.A.** Alzheimer's presenile dementia, senile dementia of Alzheimer type and Down's syndrome in middle age form an age related continuum of pathological changes. *Neuropathol. Appl. Neurobiol.,* 1984, vol. 10, 185-207
- **MITCHELL , A.J.** CSF phosphorylated tau in the diagnosis and prognosis of mild cognitive impairment and Alzheimer's disease: a meta-analysis of 51 studies. *J Neurol Neurosurg Psychiatry,* 2009, vol. 80 (9), 966-75
- **FREDERICK, J.** Pick disease. A Brief Overview. *Arch. Pathol. Lab. Med.,* 2006, vol. 130, 1063-1066
- **HANSON, J.C. ; LIPPA, C.F.** Lewy body dementia. *Int Rev Neurobiol.,* 2009, vol. 84, 215-28
- **LOVE, S.** Neurofibrillary tangles in Niemann-Pick disease type C. *Brain,* 1985, vol. 118, 119-129
- **FERRER, I.** Argyrophilic grain disease. *Brain,* 2008, vol. 131 (6), 1416-32
- **SNOWDEN, J.** Frontotemporal lobar degeneration: clinical and pathological relationships. *Acta Neuropathol.,* 2007, vol. 114 (1), 31-8
- **HUTTON, M.** Association of missense and 50-splice-site mutations in tau with the inherited dementia FTDP-17. *Nature,* 1998, vol. 393, 702-705
- **SPINA, S.** The tauopathy associated with mutation +3 in intron 10 of Tau: characterization of the MSTD family. *Brain,* 2008, vol. 131 (1), 72-89
- **HOF, P.R.** Differential distribution of neurofibrillary tangles in the cerebral cortex of dementia pugilistica and Alzheimer's disease cases. *Acta Neuropathol.,* 1992, vol. 85, 23-30
- **MCKEE, A.C.** Chronic traumatic encephalopathy in athletes: progressive tauopathy after repetitive head injury. *J Neuropathol Exp Neurol.,* 2009, vol. 68 (7), 709-35
- **WILLIAMS, D.R. ; LEES, A.J.** Progressive supranuclear palsy: clinicopathological concepts and diagnostic challenges. *Lancet Neurol.,* 2009, vol. 8 (3), 270-9
- **JELLINGER, K.A.** Absence of alpha-synuclein pathology in postencephalitic parkinsonism. *Acta Neuropathol.,* 2009, vol. 118 (3), 371-9
- **YUKSEL, D.** Tau proteins in the cerebrospinal fluid of patients with subacute sclerosing panencephalitis. *Brain Dev.,* 2010, vol. 32 (6), 467-71
- **HIRANO, A.** Amyotrophic lateral sclerosis and Parkinsonism-dementia complex on Guam. Further pathologic studies. *Arch. Neurol.,* 1966, vol. 15, 35-51
- **FELICIAN, O.** Corticobasal degeneration: clinical and neuropsychological profile. *Psychol NeuroPsychiatr Vieil,* 1966, vol. 7 (2), 91-100
- **DELISLE, M.B.** Neurodegenerative disease associated with a mutation of codon 2 79 (N279K) in exon 10 of Tau protein. *Bull Acad Natl Med.,* 2000, vol. 184 (4), 799-809
- **ASUNI, A.A.** Change in tau phosphorylation associated with neurodegeneration in the ME7 model of prion disease. *Biochem Soc Trans.,* 2010, vol. 38 (2), 545-51

- **GHETTI, B.** Gerstmann—Sträussler—Scheinker disease and the Indiana kindred. *Brain Pathol.,* 1995, vol. 5, 61-75
- **SAITO, Y.** Widespread expression of alpha-synuclein and tau immunoreactivity in Hallervorden-Spatz syndrome with protracted clinical course. *J Neurol Sci.,* 2000, vol. 177 (1), 48-59
- **KIUCHI, A.** Presenile appearance of abundant Alzheimer's neurofibrillary tangles without senile plaques in the brain in myotonic dystrophy. *Acta Neuropathol.,* 1991, vol. 82, 1-5
- **DEUTSCH, S.I.** Dysregulation of tau phosphorylation is a hypothesized point of convergence in the pathogenesis of Alzheimer's disease, frontotemporal dementia and schizophrenia with therapeutic implications. *Prog Neuropsychopharmacol Biol Psychiatry,* 2006, vol. 30 (8), 1369-80
- **ANDERSON, J.M.** Abnormal tau phosphorylation in primary progressive multiple sclerosis. *Acta Neuropathol,* 2010, vol. 119 (5), 591-600
- **BALL, M.J.** Neuronal loss, neurofibrillary tangles and granulovacuolar degeneration in the hippocampus with ageing and dementia. A quantitative study. *Acta Neuropathol.,* 1977, vol. 37, 111-118
- **TOMLINSON, B.E.** Observations on the brains of non-demented old people. *J. Neurol. Sci.,* 1968, vol. 7, 331-356
- **GASPARINI, L.** Tau inclusions in retinal ganglion cells of human P301S tau transgenic mice: Effects on axonal viability. *Neurobiol Aging.,* 07 April 2009
- **LUDOLPH, A. C.** Tauopathies with parkinsonism: clinical spectrum, neuropathologic basis, biological markers, and treatment options. *Eur J Neurol.,* 2009, vol. 16 (3), 297-309
- *Bioorganic & Medicinal Chemistry Letters,* 2011, vol. 21 (6), 1724-1727
- **T.W. GREENE ; P.G.M. WUTS.** Protective Groups in Organic Chemistry. John Wiley and Sons, 1991